(19) Europäisches Patentamt / European Patent Office / Office européen des brevets

(11) **EP 4 471 800 A1**

(12) **EUROPEAN PATENT APPLICATION**
published in accordance with Art. 153(4) EPC

(43) Date of publication:
**04.12.2024 Bulletin 2024/49**

(21) Application number: **23747114.9**

(22) Date of filing: **27.01.2023**

(51) International Patent Classification (IPC):
**G16H 50/30** (2018.01)

(52) Cooperative Patent Classification (CPC):
**G16H 50/30**

(86) International application number:
**PCT/JP2023/002729**

(87) International publication number:
**WO 2023/145904 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.01.2022 JP 2022011083**

(71) Applicants:
• **DAIKIN INDUSTRIES, LTD.**
  **Osaka-shi, Osaka 530-0001 (JP)**
• **RIKEN**
  **Wako-shi, Saitama 351-0198 (JP)**

(72) Inventors:
• **HORIUCHI, IWASAKI, Miho**
  **Wako-shi, Saitama 351-0198 (JP)**

• **MORITO, Yusuke**
  **Wako-shi, Saitama 351-0198 (JP)**
• **WATANABE, Kyosuke**
  **Wako-shi, Saitama 351-0198 (JP)**
• **WATANABE, Yasuyoshi**
  **Wako-shi, Saitama 351-0198 (JP)**
• **MIZUNO, Kei**
  **Wako-shi, Saitama 351-0198 (JP)**
• **HORI, Shouta**
  **Osaka-shi, Osaka 530-0001 (JP)**
• **YANO, SAKATA, Youko**
  **Osaka-shi, Osaka 530-0001 (JP)**

(74) Representative: **Uexküll & Stolberg**
**Partnerschaft von**
**Patent- und Rechtsanwälten mbB**
**Beselerstraße 4**
**22607 Hamburg (DE)**

(54) **SYSTEM, METHOD, AND PROGRAM FOR QUANTIFYING EFFECTS OF CONDITION CHANGE PERTAINING TO SUBJECT**

(57) The present invention provides a system for quantifying the effects of a change of a condition pertaining to a subject for a prescribed period, wherein the change, which occurs for the prescribed period, includes at least a change from a first condition to a second condition. The system comprises: a reception means which receives data measured from the subject, the data including first data measured from the subject in the first condition and second data measured from the subject in the second condition; a derivation means which derives, from the received data, at least a first component that represents an accumulation of a load on the subject for the prescribed period and a second component that varies following the change; and an output means which outputs at least one of the derived first component and second component.

Processed by Luminess, 75001 PARIS (FR)

**Fig.4A**

**Description**

TECHNICAL FIELD

The present invention relates to a system, a method, and a program for quantifying effects of a change in state regarding a subject. In particular, the present invention relates to a system or the like for quantifying effects of a change occurring during a predetermined time period, including a change from a first state to a second state.

BACKGROUND ART

There is a technique for determining a state of a living body against thermal disorder from information obtained from the living body (for example, Patent Document 1). However, a method for quantitatively evaluating effects of a change on a living body has not been sufficiently established.

PRIOR ART DOCUMENT

PATENT DOCUMENT

Patent Document 1: JP-A-2017-38839

SUMMARY OF THE INVENTION

SOLUTIONS TO THE PROBLEMS

The inventors of the present invention have considered that a solution for coping with a change can be provided by quantitatively evaluating effects of a change (for example, a change in state of the environment of a subject and a change in state of the activity of a subject) on a living body, and as a result of earnest research, have developed a new system or the like for quantifying effects of a change in state regarding a subject.
The present invention provides, for example, the following items.

(Item 1)

A system for quantifying effects of a change in state during a predetermined time period regarding a subject, the change occurring during the predetermined time period including at least a change from a first state to a second state, the system including:

a reception means that receives data measured on the subject, the data including at least first data measured on the subject in the first state and second data measured on the subject in the second state;
a derivation means that derives, from the received data, at least a first component representing accumulation of a load on the subject during the predetermined time period and a second component varying following the change; and
an output means that outputs at least one of the derived first component and second component.

(Item 2)

The system according to item 1, in which the derivation means derives the first component and the second component from the received data by applying, to the received data, a mathematical model including the first component and the second component as components.

(Item 3)

The system according to item 2, in which

the mathematical model further includes a third component that does not depend on the change as a component, and
the derivation means derives the first component, the second component, and the third component from the received data by applying the mathematical model to the received data.

(Item 4)

The system according to item 3, in which the third component is based on data measured on the subject or another subject during the predetermined time period in a case where the change does not exist.

(Item 5)

The system according to any one of items 2 to 4, in which, for each component of the mathematical model, a time series variation of each component is expressed using a local variation that follows a predetermined distribution.

(Item 6)

The system according to item 5, in which the predetermined distribution includes at least one of a normal distribution, a Cauchy distribution, a binomial distribution, a Poisson distribution, a multinomial distribution, and a Bernoulli distribution.

(Item 7)

The system according to any one of items 2 to 6, in which

the reception means further receives change information defining a change from the first state to the second state, and
the second component of the mathematical model is modeled based on the change information.

(Item 8)

The system according to item 7, in which

the state regarding the subject includes a state of an environment of the subject,
a change in the state of the environment of the subject is a periodic change or a change related to an event, and
the change information includes a period of the change or a timing of the change.

(Item 9)

The system according to any one of items 1 to 8, in which

the state regarding the subject includes a state of an environment of the subject, and
a change in the state of the environment of the subject is at least one of a change in temperature, a change in humidity, a change in radiation, a change in air quality, a change in airflow.

(Item 10)

The system according to item 7 or 8, in which

the state regarding the subject includes a state of activity of the subject,
a change in the state of the activity of the subject is a periodic change or a change related to an event, and
the change information includes a period of the change or a timing of the change.

(Item 11)

The system according to any one of items 1 to 10, in which

the state regarding the subject includes a state of activity of the subject, and
a change in the state of the activity of the subject is at least one of a change in state regarding clothing, a change in state regarding working, a change in state regarding exercise, a change in state regarding sleep, a change in state regarding dietary, and a change in state regarding excretion.

(Item 12)

The system according to any one of items 1 to 11, in which

the change occurring during the predetermined time period further includes a change from the second state to a third state, and
the data further includes third data measured on the subject in the third state.

(Item 13)

The system according to items 1 to 12, in which the data includes at least one of an autonomic nervous index based on a heart rate or heart rate variability, a skin temperature, a sweating index, a blood pressure, or a psychological assessment value.

(Item 14)

The system according to any one of items 1 to 13, further including an alarm activation means that activates an alarm when the output first component and/or second component exceeds a predetermined threshold.

(Item 15)

The system according to any one of items 1 to 14, further including an estimation/prediction means that estimates or predicts an index of a load on the subject due to the change, based on at least the output first component and/or second component.

(Item 16)

The system according to item 15, in which the estimation/prediction means predicts future variations of the first component and/or the second component.

(Item 17)

The system according to any one of items 1 to 16, further including a control means that controls an environment adjustment device based on at least the output first component and/or second component.

(Item 18)

The system according to item 17, in which
when the output first component and/or second component exceeds a predetermined threshold, the control means is configured to:

determine an environment for increasing or decreasing the first component and/or the second component; and
control the environment adjustment device to achieve the environment.

(Item 18A)

The system according to any one of items 1 to 18, in which

the first component is a component that monotonically increases or monotonically decreases in each state, and
the second component is a component that varies to approach a predetermined value in each state.

(Item 19)

A system for quantifying effects of a change in state during a predetermined time period regarding a subject, the change including at least a change from a first state to a second state, the system including:

a reception means that receives data measured on the subject, the data including at least first data measured on the subject in the first state and second data measured on the subject in the second state;

a derivation means that, by applying, to the received data, a mathematical model including a first component, a second component, and a third component as components, derives the first component, the second component, and the third component from the received data; and
an output means that outputs the derived first component and/or second component.

(Item 19A)

A system according to item 19, including features recited in one or more of the items described above.

(Item 20)

A system for quantifying effects of a change in state during a predetermined time period regarding a subject, the change including at least a change from a first state to a second state, the system including:

a reception means that receives data measured on the subject, the data including at least first data measured on the subject in the first state, second data measured on the subject in the second state, and change information defining a change from the first state to the second state;
a derivation means that, by applying, to the received data, a mathematical model including a first component, a second component, and a third component as components, derives the first component, the second component, and the third component from the received data, the second component in the mathematical model being modeled based on the change information; and
an output means that outputs the derived first component and/or second component,
in which the data includes at least one of an autonomic nervous index based on a heart rate or heart rate variability, a skin temperature, a sweating index, a blood pressure, or a psychological assessment value.

(Item 20A)

A system according to item 20, including features recited in one or more of the items described above.

(Item 21)

A method for quantifying effects of a change in state during a predetermined time period regarding a subject, the change occurring during the predetermined time period including at least a change from a first state to a second state, the method including:

receiving data measured on the subject, the data including at least first data measured on the subject in the first state and second data measured on the subject in the second state;
deriving, from the received data, at least a first component representing accumulation of a load on the subject during the predetermined time period and a second component varying following the change; and
outputting at least one of the derived first component and second component.

(Item 21A)

The method according to item 21, including features recited in one or more of the items described above.

(Item 22)

A program for quantifying effects of a change in state during a predetermined time period regarding a subject, the change occurring during the predetermined time period including at least a change from a first state to a second state, the program being executed in a computer including a processor unit, the program causing the processor unit to perform processing including:

receiving data measured on the subject, the data including at least first data measured on the subject in the first state and second data measured on the subject in the second state;
deriving, from the received data, at least a first component representing accumulation of a load on the subject during the predetermined time period and a second component varying following the change; and
outputting at least one of the derived first component and second component.

(Item 22A)

The program according to item 22, including features recited in one or more of the items described above.

ADVANTAGES OF THE INVENTION

According to the present invention, it is possible to provide a system and the like for quantifying effects of a change in state regarding a subject.

BRIEF DESCRIPTION OF THE DRAWINGS

Fig. 1 is a diagram schematically illustrating an example of informing a subject of effects of a change on the subject using components derived by a technique of the present invention.
Fig. 2 is a diagram schematically illustrating an example of controlling a surrounding environment of a subject using components derived by the technique of the present invention.
Fig. 3 is a diagram schematically illustrating an example of estimating an amount of a current load or predicting an amount of a future load on a subject using components derived by the technique of the present invention.
Fig. 4A is a diagram illustrating an example of a configuration of a system 100 for quantifying effects of a change in state during a predetermined time period regarding a subject.
Fig. 4B is a diagram illustrating an example of a configuration of a system 100', which is another embodiment of the system 100 for quantifying effects of a change in state during a predetermined time period regarding a subject.
Fig. 4C is a diagram illustrating an example of a configuration of a system 100'', which is another embodiment of the system 100 for quantifying effects of a change in state during a predetermined time period regarding a subject.
Fig. 5 is a flowchart illustrating an example of a process 500 in the system 100 for quantifying the effects of a change in state during a predetermined time period regarding a subject.
Fig. 6A is a diagram illustrating a result of Example 1.
Fig. 6B is a diagram illustrating a result of Example 1.
Fig. 6C is a diagram illustrating a result of Example 1.
Fig. 6D is a diagram illustrating a result of Example 1.
Fig. 6E is a diagram illustrating a result of Example 1.
Fig. 7A is a diagram illustrating a result of Example 2.
Fig. 7B is a diagram illustrating a result of Example 2.
Fig. 7C is a diagram illustrating a result of Example 2.
Fig. 7D is a diagram illustrating a result of Example 2.
Fig. 7E is a diagram illustrating a result of Example 2.
Fig. 8 is a diagram illustrating a result of Example 3.
Fig. 9 is a diagram illustrating a result of Example 4.
Fig. 10A is a diagram illustrating a result of Example 5A.
Fig. 10B is a diagram illustrating a result of Example 5A.
Fig. 11A is a diagram illustrating a result of Example 5B.
Fig. 11B is a diagram illustrating a result of Example 5B.

MODE FOR CARRYING OUT THE INVENTION

(1. New analysis method for effects of change in state regarding subject)

The inventors of the present invention have found a new analysis method for quantifying effects of a change by applying a mathematical model to data measured on a subject exposed to the change (for example, a change in state of an environment of the subject and a change in state of an activity of the subject). Typically, the mathematical model includes at least three components, and the inventors of the present invention have considered that a first component of the at least three components is a "component representing accumulation of a load on a subject", a second component of the at least three components is a "component varying following a change", and a third component of the at least three components is a "component independent of a change". In the present invention, by applying this mathematical model to data measured on a subject, it is possible to derive at least "a component representing accumulation of a load on a subject" and/or "a component varying following a change", which are components dependent on the change, and it is possible to quantify the effects of the change by these components.

In the present specification, the "component representing accumulation of a load on a subject" may be a component that is considered to accumulate and/or decline in the subject over time during a predetermined time period that is intended to derive the component representing the accumulation of the load on the subject due to the effects of the change, without directly following and varying the change in state regarding the subject. In the present specification and the claims, the "component representing accumulation of a load on a subject" is also referred to as a "first component". In a case where there is no change, there is no "first component". Therefore, in the mathematical model described above, the first component is not formulated. In a case where the change causes both accumulation and decline of the first component in the subject, the accumulating component and the declining component cancel each other, and in a case where the accumulating component and the declining component are the same, the variation of the first component may not be observed. Even in a case where the accumulating component and the declining component cancel each other, the first component is formulated in the above mathematical model.

In the present specification, the "component varying following a change" may refer to a component defined based on a change in state regarding a subject in a predetermined time period in which it is intended to derive a component representing accumulation of a load on the subject due to effects of the change in the mathematical model. In the present specification and the claims, the "component varying following a change" is also referred to as a "second component". In a case where there is no change, there is no "second component".

In one case, the first component is typically a component that monotonically increases or monotonically decreases in each state, and the second component is typically a component that varies to approach a predetermined value in each state. In this case, the first component does not converge until reaching a maximum or minimum value of possible values, while the second component converges to the predetermined value. This is a case where, when the effects in a direction in which the load is accumulated due to the change is regarded as effects in a positive direction and the effects in a direction in which the load is declined due to the change is regarded as effects in a negative direction, the effects in a certain direction (one of the positive direction and the negative direction) due to the change are exerted on the subject. This may be, for example, a case where the subject receives cyclic heat stress (that is, repetition of a high temperature load and removal of the high temperature load, or repetition of a low temperature load and removal of the low temperature load), a case where the subject receives a fatigue load for a certain period of time, a case where the subject recovers by taking a break, or the like. For example, in a case where the subject is subjected to cyclic heat stress, the first component of data representing heart rate variability measured on the subject increases monotonically during the cyclic heat stress, while the second component asymptotically approaches a predetermined maximum value during the heat stress and asymptotically approaches a predetermined minimum value when released from the heat stress. For example, in a case where a subject is subjected to a fatigue load for a certain period of time, a first component of data representing a correct answer rate of a certain task measured on the subject monotonously decreases during the fatigue load for the certain period of time, while a second component asymptotically approaches a predetermined minimum value during the fatigue load for the certain period of time. For example, in a case where the subject recovers with a break, the first component of data representing the subjective motivation measured on the subject increases monotonically while recovering with a break, while the second component asymptotically approaches a predetermined maximum while recovering with a break. However, in a case where there is a time delay before the change is reflected in the first component and/or the second component, or in a case where a noise or a measurement error is large, etc., this definition is not necessarily true, and the first component may increase and decrease during one state, and the second component may not approach a predetermined value.

Here, the monotonic increase means that a value $V_n$ of the first component at a certain time point has a relationship of $V_{n+a} \geq V_n$ with respect to a value $V_{n+a}$ at an arbitrary time point, and in a narrow sense, may mean that it has a relationship of $V_{n+a} > V_n$ ($a > 0$). The monotonic decrease means that the value $V_n$ of the first component at a certain time point has a relationship of $V_{n+a} \leq V_n$ with respect to the value $V_{n+a}$ at an arbitrary time point, and in a narrow sense, may mean that it has a relationship $V_{n+a} < V_n$ ($a > 0$). In the monotonic increase and monotonic decrease, it may change from $V_n$ to $V_{n+a}$ with a constant slope, may change exponentially, logarithmically, or stepwise, or may change differently.

Here, the predetermined value may be a known value or an unknown value. Furthermore, the predetermined value may or may not be a value unique to each state. To approach the predetermined value may be to approach the predetermined value by monotonically increasing or monotonically decreasing, or may approach the predetermined value while performing hunting. The predetermined value may be a value different from a maximum value or a minimum value of possible values.

In one case, the first component may be a component that does not vary following the change as compared with the second component among at least two components separated by applying the mathematical model, and the second component may be a component that varies following the change as compared with the first component among the at least two components separated by applying the mathematical model. For this, in the mathematical model, the first component and the second component are both components related to the change, but the second component may be formulated as a component defined based on the change, and the first component may be formulated as a component not defined based on the change. "Varying following a change" generally refers to a high correlation with the change.

In the present specification, the "component independent of a change" can refer to a component defined based on a time-series change in a predetermined time period of a subject in a case where no change occurs in the predetermined time period in an environment equivalent to the predetermined time period in which it is intended to derive a component representing accumulation of a load on the subject due to effects of the change in the mathematical model. The "equivalent environment" refers to an environment in which any one or more items of a temporal environment such as a season, a day, or a night, and a spatial environment such as indoor, outdoor, or latitude and longitude are equivalent to a steady state in a case where there is no change in an environment in the predetermined time period. Furthermore, the present component may be derived from a model based on a time-series change in a time period in which a change in a group to which the subject belongs does not occur, which is measured in advance. The group to which the subject belongs refers to a group in which any one or more attributes such as sex, age, work load, work content, and living environment are equivalent to those of the subject. In the present specification and the claims, the "component independent of a change" is also referred to as a "third component".

A specific mathematical model may be a state space model constructed by an observation equation formulated as a linear sum of the first component, the second component, and the third component, and a state equation in which a time-series variation of each component is formulated.

In the present technique, from data measured on a subject (for example, data related to a physical condition of the subject and/or data related to a mental condition of the subject), a component representing the accumulation of a load on the subject and/or a component varying following the change and/or a component independent of the change. Preferably, in the present technique, from the data measured on the subject, at least a component representing the accumulation of the load on the subject can be derived. More preferably, in the present technique, from the data measured on the subject, at least a component representing the accumulation of the load on the subject and a component varying following the change can be derived. More preferably, in the present technique, from the data measured on the subject, at least a component representing the accumulation of the load on the subject, a component varying following the change, and a component independent of the change can be derived.

Each component derived by the present technique can be used in a variety of applications.

In one example, at least one of the components derived by the present technique can be used to inform the subject of the effects of the change on the subject. For example, the component representing the accumulation of the load on the subject can be used to inform the subject that the accumulation of the load on the subject has exceeded a predetermined threshold. For example, the component varying following the change can be used to inform the subject that a sudden change has occurred in the physical or mental condition of the subject. Note that, in the present specification, "exceeding a predetermined threshold" means passing the predetermined threshold, and includes both (1) a case where a value smaller than the predetermined threshold becomes larger than the predetermined threshold and (2) a case where a value larger than the predetermined threshold becomes smaller than the predetermined threshold.

Hereinafter, embodiments of the present invention will be described with reference to the drawings.

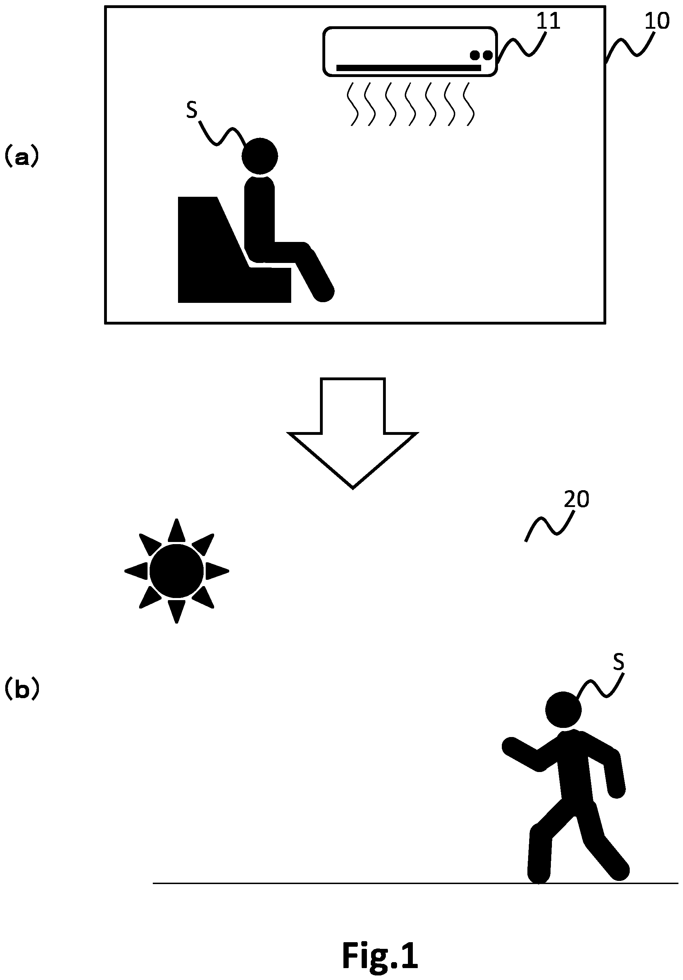

Fig. 1 schematically illustrates an example where components derived by the present technique are used to inform a subject of effects of a change on the subject. In the example illustrated in Fig. 1, a case where a subject S in a room 10 goes to an outdoor area 20 will be described as an example. An air conditioning device 11 is installed in the room 10, and a temperature of the room 10 is set to be lower than that of the outdoor area 20 by the air conditioning device 11.

The subject S changes from a state of being in the room 10 as illustrated in Fig. 1(a) to a state of being in the outdoor area 20 as illustrated in Fig. 1(b). At this time, the subject S experiences a temperature change from a room temperature of the room 10 to an air temperature of the outdoor area 20.

In the present technique, the effects of the temperature change are analyzed using data (for example, a heart rate) measured on the subject S in the room 10 and data (for example, a heart rate) measured on the subject S in an outdoor area. That is, according to the present technique, a component representing accumulation of a load on the subject, a component varying following a change, and/or a component independent of a change, which is caused by the temperature change, is derived.

For example, when a component representing the accumulation of the load on the subject exceeds a predetermined threshold due to the temperature change, a terminal device (for example, a smartphone) of the subject S or a wearable device (for example, a smart watch) worn by the subject S sounds an alarm. As a result, the subject S can know that the load is accumulated in the subject due to the temperature change, and can take measures.

For example, when a change amount of a component varying following a change exceeds a predetermined threshold due to the temperature change, a terminal device (for example, a smartphone) of the subject S or a wearable device (for example, a smart watch) worn by the subject S sounds an alarm. As a result, the subject S can know that the data (for example, a heart rate) measured on the subject rapidly changes due to the temperature change, and can take measures.

In another example, at least one of the components derived by the present technique can be used to control the ambient environment of the subject in view of the effects of the change on the subject. For example, the component representing the accumulation of the load on the subject can be used to control the surrounding environment of the subject (for example, to control an air conditioning device, and the like) so as to reduce the load on the subject when the accumulation of the load on the subject exceeds a predetermined threshold. For example, the component varying following the change can be used to control the surrounding environment of the subject (for example, to control of an air conditioning device, to control an aromatic device, to control an oxygen concentrator, and the like) so as to stabilize the physical or mental condition of the subject when an abrupt change occurs in the physical or mental condition of the subject.

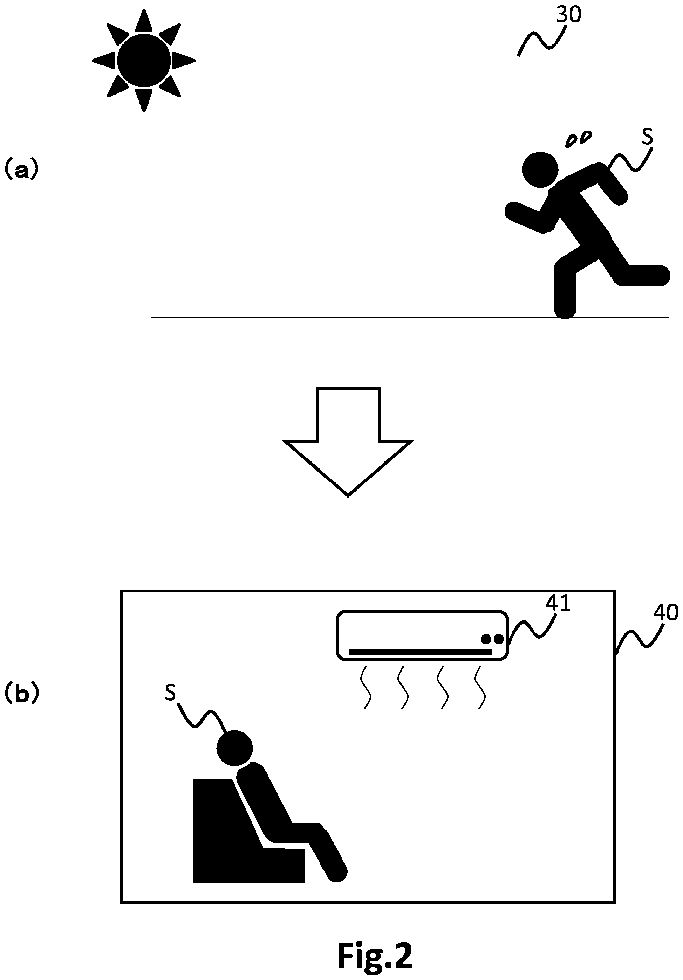

Fig. 2 schematically illustrates an example of controlling a surrounding environment of a subject using components derived by the present technique. In the example illustrated in Fig. 2, a case where a subject S exercising in an outdoor area 30 enters a room 40 and relaxes will be described as an example. An air conditioning device 41 is installed in the room 40, and the room 40 is set to a temperature slightly lower than that of the outdoor area 30 by the air conditioning device 41.

As illustrated in Fig. 2(a), the subject S changes from a state of being in the outdoor area 30 to a state of being in the room 40 as illustrated in Fig. 2(b). At this time, the subject S experiences a temperature change from an air temperature of the outdoor area 30 to a room temperature of the room 40.

Moreover, the subject S changes from a state of exercising as illustrated in Fig. 2(a) to a state of relaxing as illustrated in Fig. 2(b). At this time, the subject S experiences a change in state regarding the exercise such as the presence or absence of the exercise.

In the present technique, the effects of the temperature change and the effects of the change in state regarding the exercise are analyzed using data (for example, a heart rate) measured on the subject S exercising in the outdoor area 30 and data (for example, a heart rate) measured on the subject S relaxing in the room 40. That is, according to the present technique, a component representing accumulation of a load on the subject, a component varying following a change, and/or a component independent of a change, which is caused by the temperature change and the change in state regarding the exercise, is derived.

For example, when the component representing the accumulation of the load on the subject exceeds a predetermined threshold due to the temperature change and the change in state regarding the exercise, the air conditioning device 41 illustrated in Fig. 2(b) is driven so as to have a temperature that reduces the load on the subject. As a result, the load on the subject S can be reduced.

In another example, at least one of the components derived by the present technique can be used to estimate how much an index of a load on the subject (for example, a load amount or a load level) is currently and/or to predict how much an index of a load on the subject due to a change will be in the future due to the effects on the subject due to the change. For example, the index of the estimated current load and/or the index of the predicted future load may be utilized to provide a recommendation to the subject to facilitate behavioral changes of the subject.

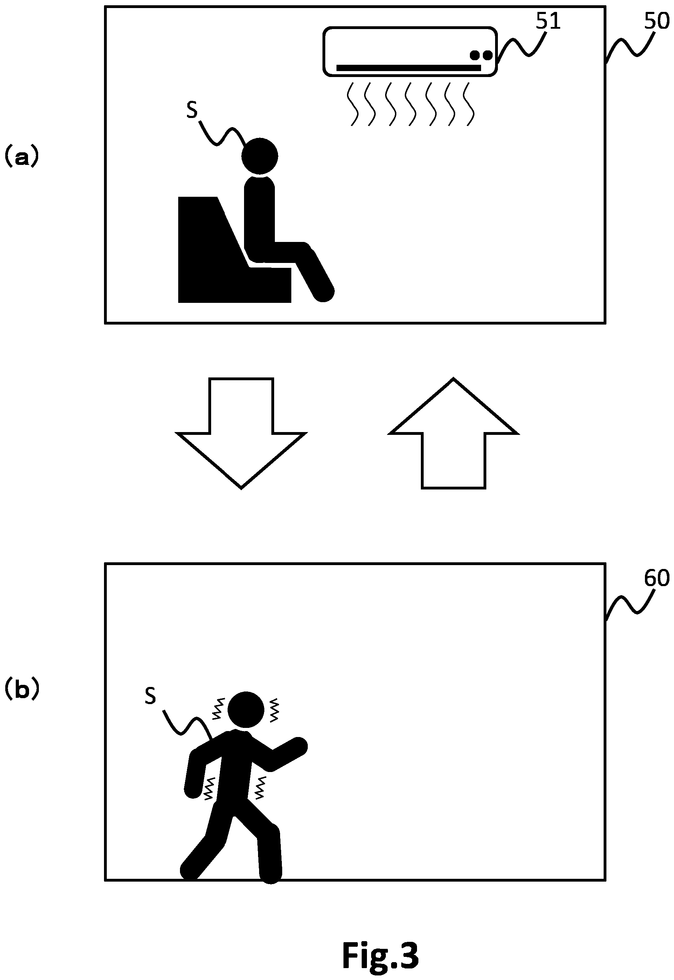

Fig. 3 schematically illustrates an example of using the components derived by the present technique to estimate an index of a current load on a subject or predict an index of a future load. In the example illustrated in Fig. 3, an example in which a subject S goes back and forth between a room 50 and a room 60 will be described. An air conditioning device 51 is installed in the room 50, and a temperature of the room 50 is set higher than that of the room 60 by the air conditioning device 51. An air conditioning device is not installed in the room 60, and the temperature is close to the temperature in an outdoor area in winter. For example, the room 50 may be a living room and the room 60 may be a corridor.

As illustrated in Fig. 3(a), the subject S changes from a state of being in the room 50 to a state of being in the room 60 as illustrated in Fig. 3(b). Then, as illustrated in Fig. 3(b), the subject changes from the state of being in the room 60 to the state of being in the room 50 as illustrated in Fig. 3(a). This process is repeated. At this time, the subject S will experience repeated temperature changes from the room temperature of the room 50 to the room temperature of the room 60 and from the room temperature of the room 60 to the room temperature of the room 50.

In the present technique, the effects of the repeated temperature changes are analyzed using data (for example, a heart rate) measured on the subject S in the room 50 and data (for example, a heart rate) measured on the subject S in the room 60. That is, the present technique derives a component representing accumulation of a load on the subject, a component varying following the change, and/or a component independent of the change, which is caused by the repeated temperature changes.

For example, the repeated temperature changes and a change of state regarding the exercise may estimate the current load of the subject and provide the subject S with a solution for reducing the current load. For example, a suitable room temperature of the room 50, a suitable stay time in the room 50, and a suitable action such as drinking a warm drink can be recommended to the subject S. The recommendation can be presented, for example, to a terminal device (for example, a smartphone) used by the subject S.

For example, the future load amount of the subject is predicted by the repeated temperature changes and the change of state regarding the exercise, and a solution for reducing the future load amount can be provided to the subject S. For example, a suitable room temperature of the room 50, a suitable stay time in the room 50, and a suitable action such as drinking a warm drink can be recommended to the subject S. The recommendation can be presented, for example, to a terminal device (for example, a smartphone) used by the subject S.

The above-described examples are merely examples using the present technique, and the present technique can be applied to any other examples. For example, it is possible to estimate a load applied to an employee by applying the present technique to data measured on the employee when the employee moves to various places on the company's work.

For example, the present technique can be applied to evaluating a change in air quality such as house dust, pollen, $CO_2$, and volatile substances (for example, volatile organic compounds (VOC) such as formaldehyde and toluene) in addition to or instead of a change related to heat such as temperature, humidity, airflow, and radiation. For example, in a room having high airtightness, repeated changes in $CO_2$ concentration occur such that the $CO_2$ concentration increases in a case where there is a person, the room is ventilated and the $CO_2$ concentration decreases when there is a person going in and out from the room, and the $CO_2$ concentration increases again when there is no person going in and out. While such changes may not have an immediate effect, the repeated changes can gradually build up a load and lead to fatigue and reduced productivity. By applying the present technique to the evaluation of air quality, such fatigue and reduction in productivity can be captured in advance.

The present technique can be implemented, for example, by a system for quantifying effects of a change in state during a predetermined time period regarding a subject, described below.

(2. Configuration of system for quantifying effects of change in state during predetermined time period regarding subject)

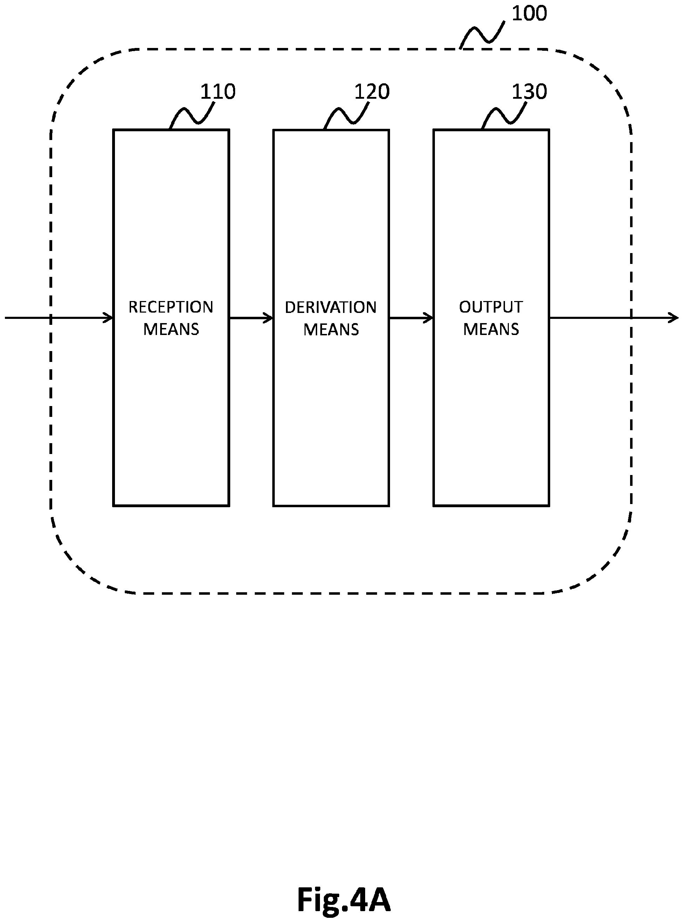

Fig. 4A illustrates an example of a configuration of a system 100 for quantifying effects of a change in state during a predetermined time period regarding a subject.

The system 100 includes a reception means 110, a derivation means 120 and an output means 130.

The system 100 is directed to a change in state regarding a subject. The state regarding the subject may be any state regarding the subject, and the state regarding the subject may be, for example, a state of an environment of the subject, a state of an activity of the subject, or both of them. The change in state regarding the subject includes a continuous variation in temperature or the like, a binary variation such as sleep or putting on and taking off clothes, and a discrete variation based on an order scale.

More specifically, the change in state of the environment of the subject includes, for example, but is not limited to, a change in temperature and humidity environment, a change in air component or air quality, a change in light environment, and a change in acoustic environment.

The change in temperature and humidity environment includes, for example, a change in temperature, a change in humidity, a change in radiation, and a change in airflow. Preferably, the change in temperature and humidity environment includes a change in temperature.

The change in air component includes, for example, a change in $CO_2$ concentration, a change in oxygen concentration, a change in hydrogen concentration, and a change in aroma.

The change in light environment includes, for example, a change in illumination.

The change in acoustic environment includes, for example, a change in music and a change in noise.

The change in state of the activity of the subject includes, for example, but is not limited to, a change in state regarding clothing, a change in state regarding behavior, and a change in state regarding sleep.

The change in state regarding behavior includes a change in state regarding working, a change in state regarding exercise, a change in state regarding dietary, and a change in state regarding excretion.

The system 100 is directed to a change that occurs during a predetermined time period. Here, the predetermined time period may be an arbitrary time period. The predetermined time period may be, for example, 1 minute, 5 minutes, 10 minutes, 30 minutes, 1 hour, 2 hours, 3 hours, 6 hours, 12 hours, 1 day, 3 days, 1 week, 2 weeks, 1 month, 2 months, 3 months, 6 months, 1 year, 2 years, 5 years, 10 years, and the like.

The change occurring during the predetermined time period may be one change (that is, a change from a first state to a second state) or a plurality of changes (that is, changes from a first state to a second state, from a second state to a third state, ···). In the case of the plurality of changes, the changes may be changes that reciprocate between two states or change between three or more states.

The change occurring during the predetermined time period may be, for example, a periodic change or a change related to an event. The periodic change includes, for example, but is not limited to, a periodic activity (for example, regularly moving in an indoor and outdoor area, sleeping and awakening, eating, and activities in which a work environment or the like changes greatly depending on the content of the activity in an indoor or outdoor area (for example, regular work in a high-temperature environment in the steel and manufacturing industry, and regular work in a cold environment (freezer or the like) in the food manufacturing industry)) by the subject. The cycle of the periodic change may be an arbitrary time period. The change related to the event includes, for example, but is not limited to, a movement in an indoor or outdoor area at an arbitrary timing, an isolated change in activity content occurring at an arbitrary timing (for example, a visit to a business operator, a coffee break during a movement in an outdoor area, a break during exercise (rehydration), and heavy labor for carrying heavy luggage in transportation or the like), excretion, exercise, and the like. The timing of the change related to the event may be any timing.

In one example, the change can be a change from a steady or resting state to an unsteady or non-resting state (for example, a state in which a load or stimulus is applied), or from an unsteady or non-resting state to a steady or resting state. In this case, the first state is one of the steady or resting state or the unsteady or non-resting state and the second state is the other of the steady or resting state or the unsteady or non-resting state.

In another example, the change may be a change from a first unsteady or non-resting state (for example, a state in which a first load or stimulus is applied) to a second unsteady or non-resting state (for example, a state in which a second load or stimulus is applied). In this case, the first state is the first unsteady or non-resting state, and the second state is the second unsteady or non-resting state. The change from the first unsteady or non-resting state to the second unsteady or non-resting state can also be considered to be a combination of the change from the first unsteady or non-resting state to a steady or resting state and the change from the steady or resting state to the second unsteady or non-resting state.

The reception means 110 is configured to receive data measured on the subject. The reception means 110 can receive the data measured on the subject in an arbitrary manner. For example, the reception means 110 may directly receive data from a measurement device that measures data from the subject, or may indirectly receive data. In the case of indirectly receiving data, for example, the reception means 110 can receive data from a terminal device (for example, a smartphone, a tablet, a desktop computer, a laptop computer, or the like) that has received the data from the measurement means. For example, the reception means 110 may receive data wirelessly or may receive data by wire. The reception means 110 may, for example, receive data read from a storage medium that stores data.

The data measured on the subject can be any data that can be measured on the subject. The data measured on the subject is intended to include not only data measured directly from the subject but also data obtained by processing data measured directly from the subject.

The data measured on the subject may be, for example, a body-related index related to a physical condition of the subject, a mind-related index related to a mental condition of the subject, or both of these. The data measured on the subject may be, for example, an index representing the performance of the subject. The performance includes physical and mental performance. The index representing the physical performance can be, for example, an index representing instantaneous force, endurance, jumping force, throwing force, or the like. The index representing the mental performance may be, for example, an index representing results for a certain task.

The body-related index includes, for example, but is not limited to, a heart beat related index related to a heart rate, a blood pressure/blood flow related index, a body temperature related index related to a body temperature, a sweating related index related to sweating, an electroencephalogram related index related to an electroencephalogram, and a respiration related index related to respiration.

The heart beat related index includes, for example, a heart rate (HR), a high frequency (HF), a low frequency (LF), LF/HF, total power (TP), and a pulse wave. In a preferred embodiment, the heart beat related index may be at least one of the HR, HF, and LF/HF. The HR, HF, LF, and LF/HF can be measured, for example, by an electrocardiograph.

The HF is a power spectrum in a frequency band of about 0.15 to 0.4 Hz, and this value represents an activity of parasympathetic (vagus) nerve. The LF is a power spectrum in a frequency band of about 0.04 to 0.15 Hz, and this value mainly represents an activity of (vasomotor) sympathetic nerve. The LF/HF represents a balance between an activity of sympathetic nerves and an activity of parasympathetic nerves. The LF/HF generally indicates superior sympathetic nervous activity when the numerical value is high, and indicates superior parasympathetic nervous activity when the numerical value is low. The HF, LF, and LF/HF can be calculated by frequency analysis from, for example, heart beat intervals measured by an electrocardiograph. That is, it is possible to calculate a low frequency component (LF) in a frequency band of about 0.04 to 0.15 Hz, a high frequency component (HF) in a frequency band of about 0.15 to 0.4 Hz, and a ratio (LF/HF) of the low frequency component/the high frequency component by performing frequency analysis (spectrum analysis) on heart rate variability (variation in R-R interval) using a technique such as Fourier transform, wavelet transform, or a maximum entropy method (MEM method). The HF, LF, and LF/HF can be calculated, for example, using other technologies known in the art.

The blood pressure/blood flow related index includes, for example, systolic blood pressure, diastolic blood pressure, peripheral blood flow, large blood vessel flow, pulse pressure, and average blood pressure. In a preferred embodiment, the blood pressure/blood flow related index may be at least one of the systolic blood pressure and the diastolic blood pressure. The blood pressure/blood flow related index can be measured by a sphygmomanometer.

The body temperature related index includes, for example, a body surface temperature and a core body temperature.

The body surface temperature may be, for example, a weighted average of skin temperatures at a plurality of points on a body surface. The body surface temperature can be measured by, for example, a skin thermometer. The core body temperature is a temperature inside the body. The core body temperature can be measured by, for example, an axillary thermometer, an intra-ear thermometer, an oral thermometer, or the like.

The sweating related index includes, for example, a sweating index.

The sweating index is a physical quantity that changes by sweating on a skin surface. Examples of the sweating index include, but are not limited to, electro dermal activity (EDA) such as skin potential activity (SPA) and skin conductance change (SC), an amount of transpiration of skin moisture, and an amount of sweating. The sweating index can be measured by, for example, an electrodermal monitor, a sweating meter, or the like.

The electroencephalogram related index includes, for example, an electroencephalogram waveform. The electroencephalogram waveform can be measured, for example, by an electroencephalograph.

The respiration related index includes, for example, a respiration rate, a depth of respiration, a respiratory volume, an oxygen concentration in exhaled air, and a carbon dioxide concentration in exhaled air. The respiration rate can be measured, for example, based on a moving image obtained by imaging the subject. The oxygen concentration in exhaled air and the carbon dioxide concentration in exhaled air can be measured, for example, by a breath analyzer.

The mind-related index is, for example, an index based on a subjective evaluation. The subjective evaluation includes, for example, but is not limited to, an evaluation related to fatigue, an evaluation related to thermal sensation, an evaluation related to comfort, an evaluation related to sleepiness, and an evaluation related to autonomic ataxia symptoms. The evaluation related to the autonomic ataxia symptoms is, for example, an evaluation of the presence or absence of subjective symptoms of the autonomic ataxia symptoms such as dizziness, headache, and hot flash. In a preferred embodiment, the mind-related index includes an index based on the evaluation related to fatigue.

The index based on a subjective evaluation may be measured by a questionnaire, a semantic differential method (SD method), or a visual analogue scale (VAS method).

The data received by the reception means 110 is data measured on the subject during a predetermined time period, and includes at least first data measured on the subject in the first state and second data measured on the subject in the second state. In a case where changes occur a plurality of times during the predetermined time period, the data received by the reception means 110 may include data measured on the subject before and after each of the changes. For example, in a case where changes occur a plurality of times from the first state to the second state, from the second state to the third state, from the third state to the fourth state, ···, the reception means 110 receives first data measured on the subject in the first state, second data measured on the subject in the second state, third data measured on the subject in the third state, fourth data measured on the subject in the fourth state, •••.

The data received by the reception means 110 is passed to the derivation means 120.

The reception means 110 can further receive change information in addition to the data measured on the subject. The change information is information that defines a change, and can define a change that occurs during a predetermined time period. The change information may define at least one of a change period or a change timing, a state value or state quantity before the change, and a state value or state quantity after the change. For example, in a case where a change occurring during a predetermined time period is a temperature change from the first state (high temperature state) to the second state (low temperature state), the change information includes a timing of the change from the first state to the second state, an air temperature in the first state, and an air temperature in the second state.

The change information received by the reception means 110 is also passed to the derivation means 120.

The derivation means 120 is configured to derive at least a first component and a second component from the data received by the reception means 110.

In an embodiment, the derivation means 120 can derive at least the first component and the second component by applying a mathematical model to the data received by the reception means 110. Here, the mathematical model includes at least the first component and the second component as components. Preferably, the mathematical model may include the first component, the second component, and a third component as components. The mathematical model may include other components in addition to the components described above. In a preferred embodiment, the mathematical model may be a state-space model used in Bayesian statistical modeling.

The derivation means 120 can derive at least the first component and the second component from the received data on the assumption that the data received by the reception means 110 has the first component and the second component of the mathematical model. In a preferred embodiment, the derivation means 120 can derive the first component, the second component, and the second component from the received data assuming that the data received by the reception means 110 has the first component, the second component, and the third component of the mathematical model.

For each component of the mathematical model, a time series variation of each component can be expressed using a local variation that follows a predetermined distribution. In one example, for a first component $Load_t$ at a certain time t, assuming that an error term is $\varepsilon_{Load,t}$, a time series variation of the first component can be expressed as a second order difference equation:

$$Load_t - Load_{t-1} = Load_{t-1} - Load_{t-2} + \varepsilon_{Load,t-2},$$

and by rearranging this, the first component is expressed as

$$Load_t = 2Load_{t-1} - Load_{t-2} + \varepsilon_{Load,t-2}.$$

Similarly, for a second component Environment (hereinafter, abbreviated as "Env"), assuming that an error term is $\varepsilon_{Env,t}$, a time series variation of the second component can be expressed as a second order difference equation:

$$Env_t - Env_{t-1} = Env_{t-1} - Env_{t-2} + \varepsilon_{Env,t-2},$$

and by rearranging this, the second component is expressed as

$$Env_t = 2Env_{t-1} - Env_{t-2} + \varepsilon_{Env,t-2}.$$

For example, in a case where the second component $Env_t$ periodically varies in a period N, the second component $Env_t$ can be expressed as

[Math 1]

$$Env_t = -\sum_{t=1}^{N-1} Env_t + \varepsilon_{Env,t}$$

Similarly, for a third component Base, assuming that an error term is $\varepsilon_{Base,t}$, a time series variation of the third component can be expressed as a second order difference equation:

$$Base_t - Base_{t-1} = Base_{t-1} - Base_{t-2} + \varepsilon_{Base,t-2},$$

and by rearranging this, the third component is expressed as

$$Base_t = 2Base_{t-1} - Base_{t-2} + \varepsilon_{Base,t-2}.$$

The local variation may follow a predetermined distribution. The predetermined distribution includes, for example, at least one of a normal distribution, a Cauchy distribution, a binomial distribution, a Poisson distribution, a multinomial distribution, and a Bernoulli distribution. The distribution of the local variation may be determined depending on the nature of the variation. For example, in a case where the variation is a continuous variation, the local variation may follow a normal distribution, and may be expressed as normal($\mu$, $\sigma$) as a mean value $\mu$ and a standard deviation $\sigma$. For example, in a case where the continuous variation includes a steep variation, the local variation may follow a Cauchy distribution and may be expressed as cauchy($\mu$, $\sigma$) as a mean value $\mu$ and a standard deviation o.

For example, when the local variation of the first component follows the Cauchy distribution, the first component $Load_t$ at a certain time t may be expressed as

$$Load_t = 2Load_{t-1} - Load_{t-2} + \varepsilon_{Load}$$

$$\varepsilon_{Load} \sim cauchy(0, \sigma_{Load}).$$

For example, assuming that the local variation of the second component follows a normal distribution, the periodic second component $Env_t$ at a certain time t may be expressed as follows.

[Math 2]

$$Env_t = -\sum_{t=1}^{N-1} Env_t + \varepsilon_{Env,t}$$
$$\varepsilon_{Env} \sim normal(0, o_{Env})$$

For example, assuming that the local variation of the third component follows a normal distribution, the first component $Base_t$ at a certain time t may be expressed as

$$Base_t = 2Base_{t-1} - Base_{t-2} + \varepsilon_{Base,t-2}$$

$$\varepsilon_{Base} \sim \texttt{normal}(0, \ \sigma_{Base}).$$

The second component of the mathematical model may be modeled based on the change information received by the reception means 110. For example, in a case where the change information indicates that the change is a periodic change, the second component may be modeled to represent the periodic change. Specifically, in a case where the change information indicates that the change is a change that periodically varies with period N, the second component may be modeled as described above as [Math. 1]. For example, in a case where the change information indicates that the change is a change related to an event, the second component may be modeled to represent the change related to the event.

The third component of the mathematical model may be modeled based on data previously measured on the subject at a predetermined time period in a case where there is no change. Alternatively, in addition to this or instead of this, the third component of the mathematical model can be modeled based on data previously measured on another subject during a predetermined time period in a case where there is no change. The another subject preferably has similar characteristics as the subject. For example, the another subject may be the same sex or the same age as the subject.

In the above example, it has been described that the state space model used in the Bayesian statistical modeling is used as the mathematical model, but the mathematical model is not limited to the state space model. Any mathematical model may be utilized as long as at least the first component and the second component can be derived from the received data.

The output means 130 is configured to output the component derived by the derivation means 120. The output means 130 can output, for example, at least one of the first component, the second component, and the third component derived by the derivation means 120. Preferably, the output means 130 outputs at least one of the first component and the second component derived by the derivation means 120. A mode in which the output means 130 outputs the derived component is not limited. The output means 130 can output the derived component in any manner.

The output means 130 can output, for example, the derived component to the outside of the system 100 by transmitting the component via a network. At this time, the type of the network is not limited. The network may be, for example, the Internet or a LAN.

The output means 130 can output the derived component to the outside of the system 100, for example, by storing the derived component in a storage medium. At this time, the type of the storage medium is not limited.

For example, the output means 130 may output the derived component to a display screen, may output the derived component by audio, or may output the derived component by printing the derived component on paper.

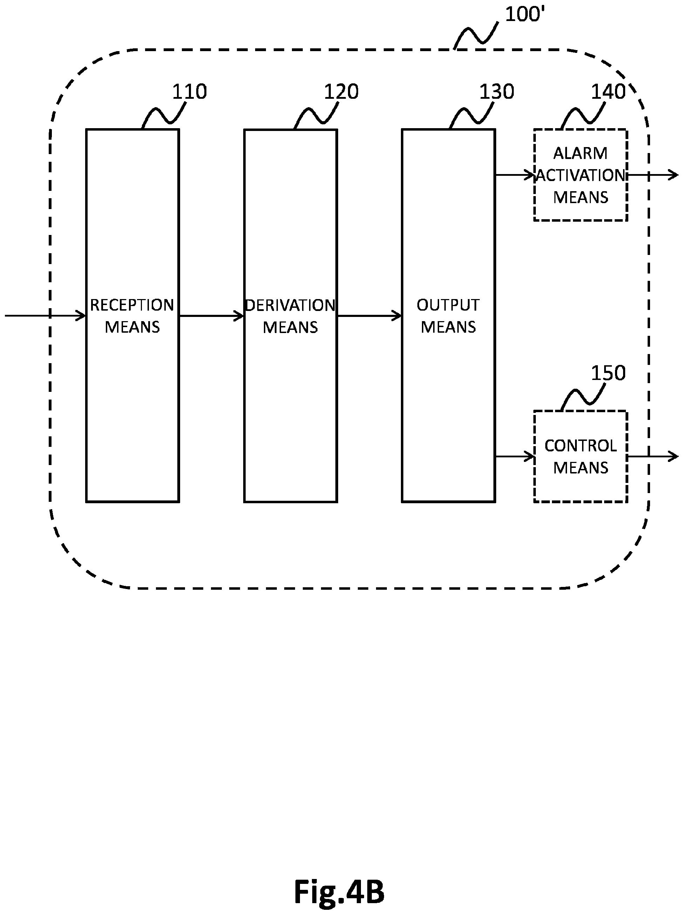

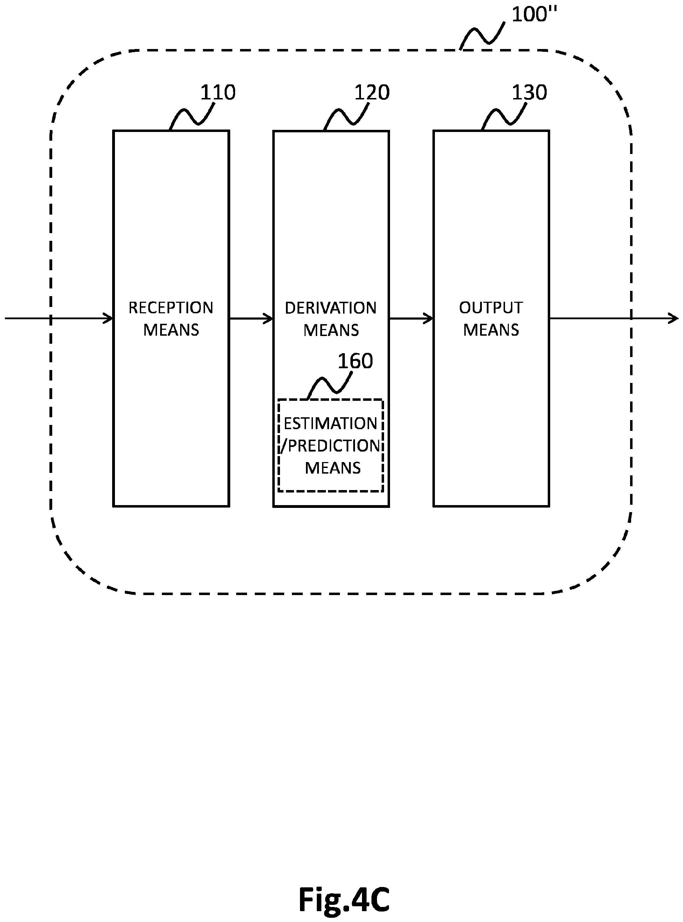

The output by the output means 130 can be used for any application. For example, it is possible to determine whether or not the output component exceeds a threshold, and perform a predetermined action in a case where it is determined that the output component exceeds the threshold. Examples of the predetermined action include, but are not limited to, activating an alarm (for example, alarm activation to the subject himself/herself, alarm activation to a person other than the subject (for example, a family member, a doctor, a nurse, a care worker, and the like)), controlling an environment adjustment device, accumulating information in a storage unit (for example, a cloud server connected via a network), and displaying on a display unit of a terminal device. For example, other information may be estimated or predicted based on the output component. The other information includes, for example, but is not limited to, information representing a current load index (for example, a load amount or a load level) of the subject, information representing a future load index (for example, a load amount or a load level) of the subject, information indicating an environment (for example, a temperature and humidity environment, an air component, a light environment, and an acoustic environment) in which the load of the subject can be reduced, information indicating an action (for example, putting on and taking off clothes, exercise, and sleep) in which the load of the subject can be reduced, information indicating an item (for example, a pocket furnace, a cooling sensation sheet, an ice pillow, a hot water bottle, and the like) in which the load of the subject can be reduced, and the like. For example, the output by the output means 130 can be utilized in particular for activating an alarm to the subject and/or controlling the environment of the subject, as illustrated in Fig 4B, and/or estimating/predicting an index of a load of the subject, as illustrated in Fig 4C.

Fig. 4B illustrates an example of a configuration of a system 100', which is another embodiment of the system 100 for quantifying effects of a change in state during a predetermined time period regarding a subject. The system 100' has a similar configuration to the system 100 (reception means 110, derivation means 120, and output means 130), but the system 100' differs from the system 100 in that it further includes at least one of an alarm activation means 140 and a control means 150. In Fig. 4B, constitution elements similar to those described above with reference to Fig. 4A are denoted by the same reference signs, and a detailed description thereof will be omitted here.

The system 100' includes a reception means 110, a derivation means 120, an output means 130, and at least one of the alarm activation means 140 and the control means 150.

The reception means 110 is configured to receive data measured on the subject. The received data is passed to the derivation means 120.

The derivation means 120 is configured to derive at least a component dependent on a change from the data received by the reception means 110. The derived component is passed to the output means 130.

The output means 130 is configured to output the component derived by the derivation means 120. The output from the output means 130 can be provided to at least one of the alarm activation means 140 and the control means 150.

The alarm activation means 140 is configured to activate an alarm when the component output by the output means 130 exceeds a predetermined threshold. The alarm activation means 140 can activate an alarm, for example, when the output first component exceeds a predetermined threshold, when the output second component exceeds a predetermined threshold, or when both the output first component and the output second component exceed respective predetermined thresholds.

The predetermined threshold may be any value, but preferably may be a value determined based on a state regarding the subject. For example, in a case where a state (for example, a hot environment, a vigorous exercise state, and the like) in which a load is likely to accumulate is involved, the predetermined threshold may be lower than a case where the load is not likely to accumulate. This makes it possible to achieve alarm activation according to the state regarding the subject.

The alarm activation means 140 can activate an alarm in an arbitrary manner. For example, the alarm activation means 140 may activate an alarm by sound, light, or vibration. For example, the alarm activation means 140 may activate an alarm to the subject himself whose data was measured, or activate an alarm to a person around the subject (for example, a family member or the like of the subject).

The control means 150 is configured to control the environment adjustment device at least based on the component output by the output means 130. The environment adjustment device may be any device capable of adjusting the environment around the subject. In one example, the environment adjustment device is a device capable of adjusting, for example, a temperature and humidity environment (for example, temperature, humidity, radiation, airflow, and the like), an air component (for example, $CO_2$ concentration, oxygen concentration, hydrogen concentration, aroma, and the like), a light environment (for example, illumination), and/or an acoustic environment (for example, music, noise, and the like). Typically, the environment adjustment device may be an air conditioning device such as an air conditioner capable of adjusting a temperature and humidity environment.

In a preferred embodiment, the control means 150 can determine an environment that increases or decreases the component output by the output means 130 when the component exceeds a predetermined threshold (that is, in a case where the component becomes larger than a predetermined threshold, an environment that decreases the component is determined, or in a case where the component becomes smaller than a predetermined threshold, an environment that increases the component is determined.), and control the environment adjustment device to achieve the determined environment. The environment that increases or decreases the component may be, for example, at least one of a specific temperature and humidity environment, a specific air component, a specific light environment, and a specific acoustic environment.

In an example, in a case where the environment adjustment device is an air conditioning device, when the first component output by the output means 130 exceeds a predetermined threshold (that is, when the accumulation of the load on the subject exceeds a predetermined amount), the control means 150 can determine a temperature and humidity environment (for example, temperature and humidity) that increases or decreases the first component, and control the air conditioning device to achieve the temperature and humidity environment.

In another example, in a case where the environment adjustment device is an acoustic device capable of adjusting an acoustic environment and an aromatic device capable of adjusting an air component, the control means 150 may determine an acoustic environment (for example, music) and an air component (for example, aroma) that increase or decrease the first component when the first component output by the output means 130 exceeds a predetermined threshold (that is, when the accumulation of the load on the subject exceeds a predetermined amount), and control the acoustic device and the aromatic device to achieve the acoustic environment and the air component.

Moreover, in yet another example, in a case where the environment adjustment device is an oxygen concentrator capable of adjusting an oxygen concentration, the control means 150 can determine the oxygen concentration stabilizing a change in the second component and control the oxygen concentrator to achieve the oxygen concentration when an amount of change in the second component output by the output means 130 exceeds a predetermined threshold (that is, when a component varying following the change suddenly changes).

The predetermined threshold may be any value, but preferably may be a value determined based on a state regarding the subject. For example, in a case where a state (for example, a hot environment, a vigorous exercise state, and the like) in which a load is likely to accumulate is involved, the predetermined threshold may be lower than a case where the load is not likely to accumulate. As a result, it is possible to achieve environmental adjustment according to the state regarding the subject.

Fig. 4C illustrates an example of a configuration of a system 100'', which is another embodiment of the system 100 for quantifying effects of a change in state during a predetermined time period regarding a subject. The system 100'' has a similar configuration to the system 100 (reception means 110, derivation means 120, and output means 130), but the system 100'' differs from the system 100 in that a derivation means 120 includes an estimation/prediction means 160. In Fig. 4C, constitution elements similar to those described above with reference to Fig. 4A are denoted by the same reference signs, and a detailed description thereof will be omitted here.

The system 100'' includes a reception means 110, the derivation means 120 and an output means 130.

The reception means 110 is configured to receive data measured on the subject. The received data is passed to the derivation means 120.

The derivation means 120 is configured to derive at least a first component and a second component from the data received by the reception means 110. The derivation means 120 includes the estimation/prediction means 160.

The estimation/prediction means 160 is configured to estimate or predict an index of a load on a subject due to a change, based on at least the first component and/or the second component. The load index is an index that can quantitatively or qualitatively represent a degree of load. The load index includes, for example, a load amount quantitatively representing a degree of load in a specific tome period, a load change amount quantitatively representing a degree of a change in load per unit time, a load level qualitatively representing a degree of load in a specific time period, an amplitude indicating a width of variation in load, and the like. The load index may be absolute or relative.

In one embodiment, the estimation/prediction means 160 can estimate an index of the current load on the subject.

The index of the current load may be defined, for example, as a value based on an approximation formula of the time series variation of the component (for example, a first component and/or a second component) that depends on a change.

For example, in a case where the time series variation of the first component (Load) is approximated as in a formula below

[Math 3]

$$Load(t) = \alpha t$$

$\alpha$ corresponding to the slope can be regarded as an index of the current load. Here, $\alpha$ may be a load increase amount that quantitatively represents the degree of increase in load. The load amount during a predetermined time period $\Delta t$ can be estimated as the load increase amount $\times \Delta t$.

For example, in a case where the time series variation of the second component (Env) is approximated as in a formula below

[Math 4]

$$Env(t) = \beta \sin\left(\frac{2\pi}{4}t\right) + \gamma$$

$\beta$ corresponding to the amplitude can be regarded as an index of the current load. Here, $\beta$ may be a load level that qualitatively represents a magnitude of the load variation. The load amount during the predetermined time period $\Delta t$ can be estimated as an integral value of Env(t) at $\Delta t$.

Among the load indices, the load amount at the present time (time $t_0$) can be estimated as, for example, a value obtained by multiplying a value at or near the time $t_0$ of the component (for example, the first component and/or the second component) depending on the change or a calculated value thereof by the temporal change amount $\Delta t$. The computed value may be, for example, a nearby value or an amount of change within a given time period (for example, an increment or a decrement), a difference between nearby values or extreme values within a given time period, a nearby value or an average value within a given time period, and the like.

In another embodiment, the estimation/prediction means 160 can predict an index of a future load in addition to or instead of estimating an index of the current load. For example, the estimation/prediction means 160 may predict a future variation of a component dependent on a change (for example, a first component and/or a second component) as an index of the future load.

For example, the estimation/prediction means 160 can model the time-series variation of the first component and/or the second component with a model formula, and predict the future variation on the basis of the model formula. At this time, the model formula can be expressed using a local variation according to a predetermined distribution.

For example, the estimation/prediction means 160 models the future variation of the first component as in a formula below

$$\text{Load}_t = 2\text{Load}_{t-1} - \text{Load}_{t-2} + \varepsilon_{\text{Load}}$$

$$\varepsilon_{\text{Load}} \sim \text{normal}(0, \sigma_{\text{Load}}),$$

and models the future variations of the second component as in a formula below

[Math 5]

$$\mathrm{Env}_t = -\sum_{t=1}^{N-1} Env_t + \varepsilon_{\mathrm{Env},t}$$
$$\varepsilon_{\mathrm{Env}} \sim \mathrm{normal}(0, \sigma_{\mathrm{Env}})$$

the index of the future load can be predicted based on these models.

The index of the future load can be defined, for example, as a value based on an approximation formula of the future time series variation of the component (for example, a first component and/or a second component) that depends on a change.

For example, in a case where, based on the model described above, the future time series variation of the first component (Load) is approximated as in a formula below

[Math 6]

$$Load(t) = \alpha t$$

$\alpha$ corresponding to the slope can be regarded as an index of the future load. Here, $\alpha$ may be a load increase amount that quantitatively represents the degree of increase in future load. The load amount after the predetermined time period $\Delta t$ can be estimated as the load increase amount $\times \Delta t$.

For example, in a case where, based on the model described above, the future time series variation of the second component (Env) is approximated as in a formula below

[Math 7]

$$Env(t) = \beta \sin\left(\frac{2\pi}{4} t\right) + \gamma$$

$\beta$ corresponding to the amplitude can be regarded as an index of the future load. Here, $\beta$ may be a load level that qualitatively represents a magnitude of the future load variation. The load amount during the predetermined time period $\Delta t$ can be estimated as an integral value of $\Delta t$ of Env(t).

Among the load indices, the future load amount (time $t_1$) can be estimated as, for example, a value obtained by multiplying a value at or near the time $t_1$ calculated from the above-described model or a calculated value thereof by the temporal change amount $\Delta t$. The computed value may be, for example, a nearby value or an amount of change within a given time period (for example, an increment or a decrement), a difference between nearby values or extreme values within a given time period, a nearby value or an average value within a given time period, and the like.

The output means 130 is configured to output the component derived by the derivation means 120 and/or an index of the current or future load.

The system 100" may include at least one of an alarm activation means 140 and a control means 150, similarly to the system 100'. At this time, the alarm activation means 140 can activate an alarm based on the index of the current load and/or the index of the future load, and the control means 150 can control the environment adjustment device based on the index of the current load and/or the index of the future load.

In one embodiment, the system 100 described above may be implemented as a server device, for example. The server device can communicate with a measurement device that measures data from the subject, a local device of the subject (for example, a terminal device, an alarm activation device, an environment adjustment device, or the like). The server device may include a communication interface unit, a storage unit, and a processor unit.

The communication interface unit controls communication between the server device and the outside of the server device. The server device can communicate with the measurement device and the local device via the communication interface unit.

For example, the server device can receive data measured on the subject via the communication interface unit. In this respect, the communication interface unit may constitute the reception means 110 of the system 100.

For example, the server device can output an output from the server device to the outside of the server device via the communication interface unit. In this respect, the communication interface unit may constitute the output means 130 of the system 100.

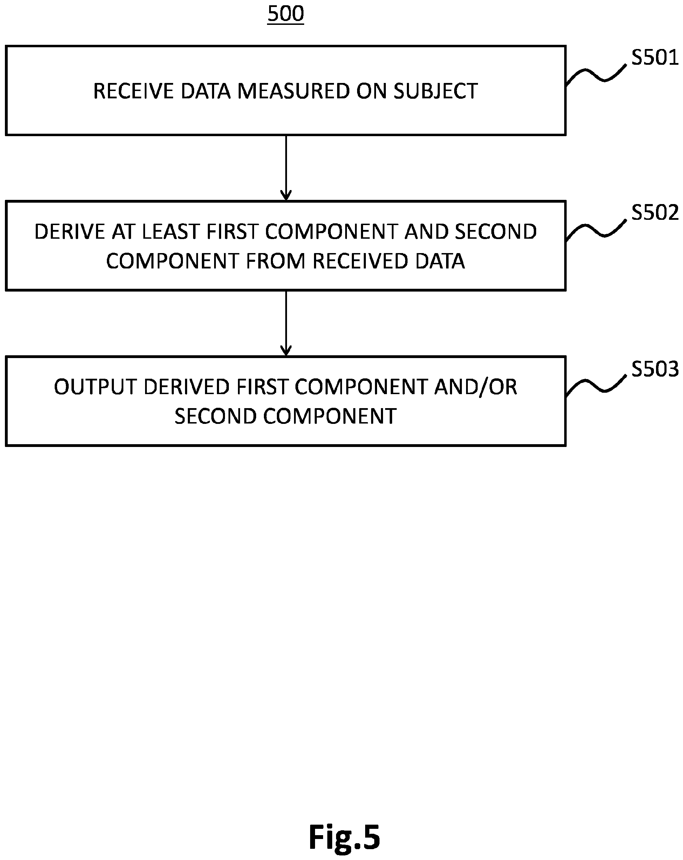

The storage unit stores a program required for execution of processing of the server device, data required for execution of the program, and the like. For example, the storage unit stores a part or all of a program (for example, a program for realizing a process illustrated in Fig. 5 to be described later) for causing the server device to perform a process for quantifying effects of a change in state during a predetermined time period regarding the subject. Here, how to store the program in the storage unit does not matter. For example, the program may be pre-installed in the storage unit. Alternatively, the program may be installed in the storage unit by being downloaded via a network. The program may be stored on a computer-readable tangible storage medium. The storage unit may be implemented by any storage means such as a memory or a database.

The storage unit may store model formulas of the first component, the second component, and the third component. The storage unit can store a plurality of model formulas specific to the change in state regarding the subject for each of the first component, the second component, and the third component. For example, the model expression may be a model expression specific to a case where the change in state regarding the subject is a periodic change, a model formula specific to a case where the change in state regarding the subject is a change related to an event, or the like. As a result, the first component, the second component, and the third component can be derived using the model formula according to the change in state regarding the subject among the plurality of stored model formulas.

The processor unit controls the operation of the entire server device. The processor unit reads a program stored in the storage unit and executes the program. As a result, it is possible to cause the server device to function as a device that executes a desired step. The processor unit may be implemented by a single processor or may be implemented by a plurality of processors. The derivation means 120 of the system 100 may be implemented by the processor unit. Moreover, the processor unit can constitute the reception means 110 and the output means 130 in that the processor unit receives the data measured on the subject from the outside of the processor unit and the processor unit outputs the derived component to the outside of the processor unit.

In another embodiment, the system 100 or system 100' or 100" described above can be implemented, for example, as a local device of the subject. The local device may be in communication with a measurement device that measures data from the subject or may be configured integrally with the measurement device that measures data from the subject. The local device may include at least a communication interface unit, a storage unit, and a processor unit.

The communication interface unit controls communication between the local device and the outside of the local device. The local device may communicate with the measurement device via the communication interface unit.

The local device of the subject may receive data measured on the subject, for example, via the communication interface unit. In this respect, the communication interface unit may constitute the reception means 110 of the system 100 or the system 100' or 100".

For example, the local device of the subject can output the output from the local device to the outside of the local device via the communication interface unit. In this respect, the communication interface unit may constitute the output means 130 of the system 100 or the system 100' or 100''.

The storage unit stores a program required for executing processing of the local device of the subject, data required for executing the program, and the like. For example, the storage unit stores a part or all of a program (for example, a program for realizing a process illustrated in Fig. 5 to be described later) for causing the local device of the subject to perform a process for quantifying effects of a change in state during a predetermined time period regarding the subject. Here, how to store the program in the storage unit does not matter. For example, the program may be pre-installed in the storage unit. Alternatively, the program may be installed in the storage unit by being downloaded via a network. The program may be stored on a computer-readable tangible storage medium. The storage unit may be implemented by any storage means such as a memory or a database.

The storage unit may store model formulas of the first component, the second component, and the third component. The storage unit can store a plurality of model formulas specific to the change in state regarding the subject for each of the first component, the second component, and the third component. For example, the model expression may be a model expression specific to a case where the change in state regarding the subject is a periodic change, a model formula specific to a case where the change in state regarding the subject is a change related to an event, or the like. As a result, the first component, the second component, and the third component can be derived using the model formula according to the change in state regarding the subject among the plurality of stored model formulas.

The processor unit controls the operation of the entire local device of the subject. The processor unit reads a program stored in the storage unit and executes the program. As a result, it is possible to cause the local device of the subject to function as a device that executes a desired step. The processor unit may be implemented by a single processor or may be implemented by a plurality of processors. The derivation means 120 of the system 100 may be implemented by the processor unit. Moreover, the processor unit can constitute the reception means 110 and the output means 130 in that the processor unit receives the data measured on the subject from the outside of the processor unit and the processor unit outputs the derived component to the outside of the processor unit.

The local device of the subject may, for example, include an alarm activation means having a function as an alarm activation device or communicate with the alarm activation device. In a case where the local device includes the alarm activation means, the processor unit determines whether or not the output exceeds a predetermined threshold, and the alarm activation means can activate an alarm in a case where it is determined that the output exceeds the predetermined threshold. The alarm activation means may constitute the alarm activation means 140 of the system 100'. Alternatively, in a case where the local device communicates with the alarm activation device, the alarm activation device may include, for example, a communication interface unit, a processor unit, and an alarm activation means. In the alarm activation device, the communication interface unit receives the output from the local device, the processor unit determines whether or not the output exceeds a predetermined threshold, and the alarm activation means can activate an alarm in a case where it is determined that the output exceeds the predetermined threshold. The alarm activation device may constitute the alarm activation means 140 of the system 100'. Alternatively, the processor unit of the local device may determine whether or not the output exceeds a predetermined threshold, and in a case where it is determined that the output exceeds the predetermined threshold, the alarm activation device may receive a control signal from the local device, and the alarm activation means may activate an alarm in response thereto. The processor unit of the local device and the alarm activation device may constitute the alarm activation means 140 of the system 100'.

The local device of the subject may be, for example, a terminal device. The terminal device may typically be a smartphone, a tablet, a desktop computer, a laptop computer, a smart watch, smart glasses, or the like. The processor unit of the local device may estimate/predict an index of the load of the subject based on the derived component. The estimated/predicted index of the load may, for example, be displayed on a display of the terminal device, or may be transmitted to another local device and used to control that local device. The processor unit of the terminal device may constitute the estimation/prediction means 160 of the system 100".

The local device of the subject may, for example, include an environment adjustment means having a function as an environment adjustment device or communicate with the environment adjustment device. In a case where the local device includes the environment adjustment means, the processor unit determines whether or not the output exceeds a predetermined threshold, and in a case where it is determined that the output exceeds the predetermined threshold, the environment adjustment means can adjust the environment around the subject. The environment adjustment means may constitute the control means 150 of the system 100'. Alternatively, in a case where the local device communicates with the environment adjustment device, the environment adjustment device may include, for example, a communication interface unit, a processor unit, and an environment adjustment means. In the environment adjustment device, the communication interface unit may receive the output from the local device, and the processor unit may control the environment adjustment means based on the output. The environment adjustment device may constitute the control means 150 of the system 100'. Alternatively, the processor unit of the local device may generate the control signal based on the output, and the environment adjustment device may receive the control signal from the local device and control the environment adjustment means according to the control signal. The processor unit of the local device and the environment adjustment device may constitute the control means 150 of the system 100'. The environment adjustment device may typically be an air conditioning device such as an air conditioner capable of adjusting a temperature and humidity environment.

In yet another embodiment, the system 100' or 100" described above can be implemented, for example, as a combination of a server device and a local device of the subject. As described above, the server device may include the communication interface unit, the storage unit, and the processor unit, and the reception means 110, the derivation means 120, and the output means 130 of the system 100' or 100'' may be configured by the communication interface and/or the processor unit of the server device.

The server device can communicate with the local device of the subject. The output from the server device may be transmitted to the local device of the subject via the communication interface unit. Here, the output from the server device can be transmitted to the local device of the subject in an arbitrary manner.

The local device of the subject may be, for example, an alarm activation device. The alarm activation device may include, for example, a communication interface unit, a processor unit, and an alarm activation means. The communication interface unit receives the output from the server device, and the processor unit determines whether or not the output exceeds a predetermined threshold, and the alarm activation device can activate an alarm in a case where it is determined that the output exceeds the predetermined threshold. The alarm activation device may constitute the alarm activation means 140 of the system 100'. Alternatively, the processor unit of the server device determines whether or not the output exceeds the predetermined threshold, and in a case where it is determined that the output exceeds the predetermined threshold, the alarm activation device receives a control signal from the server device, and the alarm activation means can activate an alarm according to the control signal. The processor unit of the server device and the alarm activation device may constitute the alarm activation means 140 of the system 100'.

The local device of the subject may be, for example, a terminal device. The terminal device may include, for example, a communication interface unit and a processor unit. In the terminal device, the communication interface unit receives the output from the server device, and the processor unit can estimate/predict the index of the load of the subject based on the output. The estimated/predicted index of the load may, for example, be displayed on a display of the terminal device or may be transmitted to other local devices and used to control that local device. The terminal device may constitute the estimation/prediction means 160 of the system 100".

The local device of the subject may be, for example, an environment adjustment device. The environment adjustment device may include, for example, a communication interface unit, a processor unit, and an environment adjustment means. In the environment adjustment device, the communication interface unit may receive the output from the server device, and the processor unit may control the environment adjustment means based on the output. The environment adjustment device may constitute the control means 150 of the system 100'. Alternatively, the processor unit of the server device may generate a control signal based on the output, and the environment adjustment device may receive the control signal from the server device, and control the environment adjustment means according to the control signal. The processor unit of the server device and the environment adjustment device may constitute the control means 150 of the system 100'. The environment adjustment device may typically be an air conditioning device such as an air conditioner capable of adjusting a temperature and humidity environment.

In the above-described example, each constitution element of the server device can be provided in the server device and the local device, respectively, but the present invention is not limited thereto. Any of the constitution elements of the server device can be provided outside the server device. For example, in a case where each of the storage unit and the processor unit is configured by separate hardware components, the hardware components may be connected via an arbitrary network. At this time, the type of the network is not limited. The hardware components may be connected via, for example, a LAN, may be wirelessly connected, or may be connected by wire. The server device is not limited to a specific hardware configuration. For example, it is also within the scope of the present invention that the processor unit is configured by an analog circuit instead of a digital circuit. For example, the database unit as the storage unit may be provided outside the server device or in the server device. The configuration of the database unit is not limited to a specific hardware configuration. For example, the database unit may be configured by a single hardware component or may be configured by a plurality of hardware components. For example, the database unit may be configured as an external hard disk device of the server device 200, or may be configured as a storage on a cloud connected via a network. The configuration of the server device is not limited to that described above as long as the function can be realized.

In the above-described example, each constitution element of the local device may be provided in the local device and the local device, respectively, but the present invention is not limited thereto. Any of the constitution elements of the local device may be provided outside the local device. For example, in a case where each of the storage unit and the processor unit is configured by separate hardware components, the hardware components may be connected via an arbitrary network. At this time, the type of the network is not limited. The hardware components may be connected via, for example, a LAN, may be wirelessly connected, or may be connected by wire. The local device is not limited to a specific hardware configuration. For example, it is also within the scope of the present invention that the processor unit is configured by an analog circuit instead of a digital circuit. The configuration of the local device is not limited to that described above as long as the function can be realized.

(4. Processing by system for quantifying effects of change in state during predetermined time period regarding subject)

Fig. 5 illustrates an example of a process 500 in the system 100 for quantifying effects of a change in state during a predetermined time period regarding a subject. The system 100' may perform similar processing.

In step S501, the reception means 110 receives data measured on the subject. The data measured on the subject is measured on the subject during a predetermined time period and includes at least the first data measured on the subject in the first state and the second data measured on the subject in the second state. In a case where changes occur a plurality of times during the predetermined time period, in step S501, the reception means 110 receives data measured on the subject before and after each of the changes. For example, in a case where changes occur a plurality of times from the first state to the second state, from the second state to the third state, from the third state to the fourth state, ···, the reception means 110 receives first data measured on the subject in the first state, second data measured on the subject in the second state, third data measured on the subject in the third state, fourth data measured on the subject in the fourth state, ···. The reception means 110 may receive the measured data each time the data is measured on the subject, or may collectively receive some or all of the data after the data is measured.

In step S501, the reception means 110 may further receive change information in addition to the data measured on the subject. The change information may be, for example, information input by the subject or information detected from the environment of the subject.

In step S502, the derivation means 120 derives at least the first component and the second component from the data received in step S501.

In one embodiment, in step S502, the derivation means 120 can derive at least the first component and the second component by applying the mathematical model to the data received in step S501. Here, the mathematical model includes at least the first component and the second component as components. Preferably, the mathematical model may include the first component, the second component, and a third component as components. The mathematical model may include other components in addition to the components described above. In a preferred embodiment, the mathematical model may be a state-space model used in Bayesian statistical modeling.

The derivation means 120 can derive at least the first component and the second component from the received data on the assumption that the data received in step S501 has the first component and the second component included in the mathematical model. In a preferred embodiment, the derivation means 120 can derive the first component, the second component, and the second component from the received data assuming that the data received in step S501 has the first component, the second component, and the third component included in the mathematical model.

In step S503, the output means 130 outputs the component derived in step S502. The output means 130 can output the derived component in any manner. The output means 130 can output, for example, the derived component to the outside of the system 100 by transmitting the component via a network. The output means 130 can output the derived component to the outside of the system 100, for example, by storing the derived component in a storage medium. For example, the output means 130 may output the derived component to a display screen, may output the derived component by audio, or may output the derived component by printing the derived component on paper.

The components output in this manner can be used for any application. For example, it is possible to determine whether or not the output component exceeds a threshold, and perform a predetermined action in a case where it is determined that the output component exceeds the threshold. Examples of the predetermined action include, but are not limited to, activating an alarm (for example, alarm activation to the subject himself/herself, alarm activation to a person other than the subject (for example, a family member, a doctor, a nurse, a care worker, and the like)), controlling an environment adjustment device, accumulating information in a storage unit (for example, a cloud server connected via a network), and displaying on a display unit of a terminal device. For example, other information may be estimated or predicted based on the output component. The other information includes, for example, but is not limited to, information representing a current load index (for example, a load amount or a load level) of the subject, information representing a future load index (for example, a load amount or a load level) of the subject, information indicating an environment (for example, a temperature and humidity environment, an air component, a light environment, and an acoustic environment) in which the load of the subject can be reduced, information indicating an action (for example, putting on and taking off clothes, exercise, and sleep) in which the load of the subject can be reduced, information indicating an item (for example, a pocket furnace, a cooling sensation sheet, an ice pillow, a hot water bottle, and the like) in which the load of the subject can be reduced, and the like.

In a case where the process 500 is performed by the system 100', after step S503, the alarm activation means 140 activates an alarm when the component output in step S503 exceeds a predetermined threshold. The alarm activation means 140 can activate an alarm, for example, when the output first component exceeds a predetermined threshold, when the output second component exceeds a predetermined threshold, or when both the output first component and the output second component exceed respective predetermined thresholds.

Alternatively, in addition to or instead of these, after step S503, the control means 150 controls the environment adjustment device based on the component output in step S503. The environment adjustment device may be any device capable of adjusting the environment around the subject. In one example, the environment adjustment device is a device capable of adjusting, for example, a temperature and humidity environment (for example, temperature, humidity, radiation, airflow, and the like), an air component (for example, $CO_2$ concentration, oxygen concentration, hydrogen concentration, aroma, and the like), a light environment (for example, illumination), and/or an acoustic environment (for example, music, noise, and the like). Typically, the environment adjustment device may be an air conditioning device such as an air conditioner capable of adjusting a temperature and humidity environment.

In a preferred embodiment, when the component output in step S503 exceeds a predetermined threshold, the control means 150 can determine an environment that increases or decreases the component, and control the environment adjustment device to achieve the determined environment. The environment that increases or decreases the component may be, for example, at least one of a specific temperature and humidity environment, a specific air component, a specific light environment, and a specific acoustic environment.

In a case where the process 500 is performed by the system 100", the estimation/prediction means 160 may estimate or predict an index of the load on the subject due to the change in step S502' instead of step S502.

In one embodiment, the estimation/prediction means 160 can estimate an index of the current load on the subject. The estimation/prediction means 160 can, for example, estimate an index of the current load on the subject based on the time series variation of the modeled first component and/or second component.

In another embodiment, the estimation/prediction means 160 can predict an index of a future load in addition to or instead of estimating an index of the current load. For example, the estimation/prediction means 160 may predict the future variation on the basis of the time series variation of the modeled first component and/or second component as an index of the future load.

The process 500 can be triggered, for example, by the occurrence of a change in state regarding the subject. For example, data may be periodically measured on the subject, and when a change occurs, the process 500 may be performed using data measured before and after the change. Alternatively, the process 500 can be performed, for example, in real-time during a predetermined time period.

In a case where the system 100 or the system 100' or the system 100" is implemented by the server device, the process 500 may be performed by the processor unit of the server device. In a case where the system 100 or the system 100' or the system 100" is implemented by the local device, the process 500 may be performed by the processor unit of the local device.

In the above-described example, it has been described that the processing of each step is performed in a specific order, but the order of the processing of each step is not limited to that described. The processing of each step can be performed in any order logically possible.

In the above-described example, it has been described that the processing of each step illustrated in Fig. 5 is realized by the processor unit and the program stored in the memory unit, but the present invention is not limited thereto. At least one of the processing of the steps illustrated in Fig. 5 may be realized by a hardware configuration such as a control circuit.

The present invention is not limited to the above-described embodiments. It is understood that the invention is to be interpreted only by the claims. It is understood from the description of the specific preferred embodiments of the present invention that a person skilled in the art can implement equivalent ranges based on the description of the present invention and the common general knowledge.

EXAMPLES

(Example 1)

(Experimental conditions)

A temperature difference test was performed on 27 subjects (13 males and 14 females) aged 22 to 58 years (mean 44.0 $\pm$ 9.4 years). The subject reciprocated between a test chamber A and a test chamber B under the following conditions. The subject stayed in each test chamber for about 20 minutes, and reciprocated between the test chamber A and the test chamber B four times. The total test time was about 180 minutes. In order to exclude the physiological effects of walking, during the experiment including during movement, the subject was in a resting state with the eyes open while sitting on a chair. The movement between rooms was performed by the assistant pushing a chair while the subject was seated on the chair. No specific task was performed on the subject. The humidity was 50% in all rooms.

[Table 1]

| Condition name | Temperature difference | Test chamber A | Test chamber B |
| --- | --- | --- | --- |
| T26-26 | 0°C | 26°C | 26°C |
| T26-31 | 5°C | 26°C | 31°C |
| T26-36 | 10°C | 26°C | 36°C |
| T21-36 | 15°C | 21°C | 36°C |

During the experiment, the following measurement items were measured.

Subjective evaluation: thermal sensation, fatigue, and autonomic ataxia
Heart rate variability: heart rate, sympathetic nerve activity, parasympathetic nerve activity, etc
Skin temperature: body surface temperature (4-point method)

(Analysis)

Analysis was performed on the measured data according to the technique of the present invention. Specifically, analysis was performed using Bayesian statistical modeling.

It was assumed that the measured data consists of a first component (Load), a second component (Environment), and a third component (Base). The measured data under each condition was defined as follows using error terms

$\varepsilon_n \sim normal(0, \sigma_n)$.

T26-26 condition (that is, the control condition): Base + $\varepsilon_1$,
Each of T26-31 condition, T26-36 condition, and T21-36 condition: Base + $Load_n$ + $Env_n$ + $\varepsilon_n$
Each component was modeled as follows:

[Math 8]

$$Base_t = 2Base_{t-1} - Base_{t-2} + \varepsilon_{Base, t-2}$$
$$\varepsilon_{Base, t} \sim normal(0, \sigma_{Base})$$
$$Load_{t, n} = 2Load_{t-1, n} - Load_{t-2, n} + \varepsilon_{Load, t-2}$$
$$\varepsilon_{Load, t} \sim cauchy(0, \sigma_{Load})$$
$$Env_{t, n} = -\sum_{t=1}^{N-1} Env_t + \varepsilon_{Env, t}$$
$$\varepsilon_{Env, t} \sim normal(0, \sigma_{Env})$$

Here, X-normal(p, $\sigma$) means that X follows a normal distribution with an average $\mu$ and a standard deviation $\sigma$, and X~cauchy($\mu$, $\sigma$) means that X follows a cauchy distribution with an average $\mu$ and a standard deviation $\sigma$. t represents a time point, and N represents the number of time points (for example, six time points) for one cycle of the temperature change.

The above model formula is modeled assuming a second-order difference expression ($x_t - x_{t-1} = x_{t-1} - x_{t-2}$).

(Results)

(1. LF/HF)

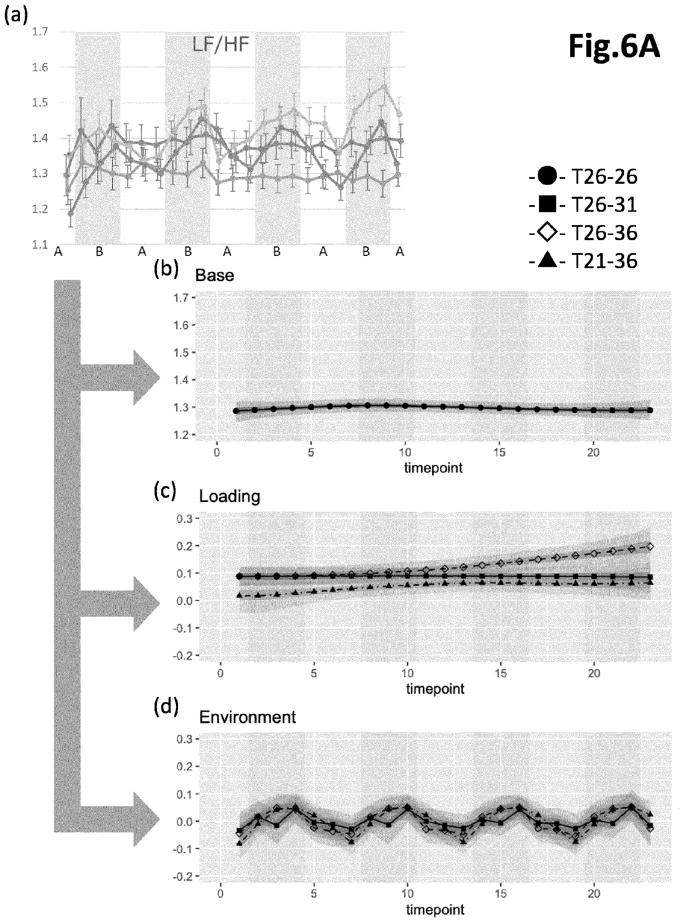

Fig. 6A illustrates results for the LF/HF index obtained from heart rate variability. Fig. 6A(a) is a graph illustrating data before analysis, and Figs. 6A(b) to 6A(d) are graphs illustrating each derived component. Fig. 6A(b) is a graph illustrating a third component (Base) that does not depend on a change, Fig. 6A(c) is a graph illustrating a first component (Loading) representing accumulation of a load, and Fig. 6A(d) is a graph illustrating a second component (Environment) that varies following the change. A black circle (•) indicates the result under the T26-26 condition, a black square (■) indicates the result under the T26-31 condition, a white diamond (◇) indicates the result under the T26-36 condition, and a black triangle (·) indicates the result under the T21-36 condition. Light grey represents the 95% Bayes credit interval and dark grey represents the 50% Bayes credit interval.

As can be seen from Fig. 6A(c), accumulation of load and fatigue on the autonomic nerve can be expressed in the first component. A degree of the accumulation of the load is

$$T26-36 \leq T26-31 < T26-36.$$

It can be seen that the load tends to accumulate more in the T21-36 condition than in the T26-36 condition where the temperature difference is the largest. Rather, it can be seen that the load under the T21-36 condition where the temperature difference is the largest falls within the range of the T26-31 condition where the temperature difference is small. This is presumed to be because the accumulation of the load is reset by entering the low temperature condition of 21°C after the high temperature condition of 36°C. The degree of accumulation of such a load cannot be found from the data before analysis illustrated in Fig. 6A(a). By the technique of the present invention, the degree of the accumulation of the load can be visualized because the first component can be distinguished from the second component and the third component and derived.

As can be seen from Fig. 6A(d), the effects of the environment for each cycle are expressed in the second component. Such effects of the environment for each cycle could not be clearly found from the data before analysis illustrated in Fig. 6A(a). By the technique of the present invention, it is possible to distinguish and derive the second component from the first component and the third component, and thus, it is possible to visualize the effects of the environment for each cycle.

(2. HR)

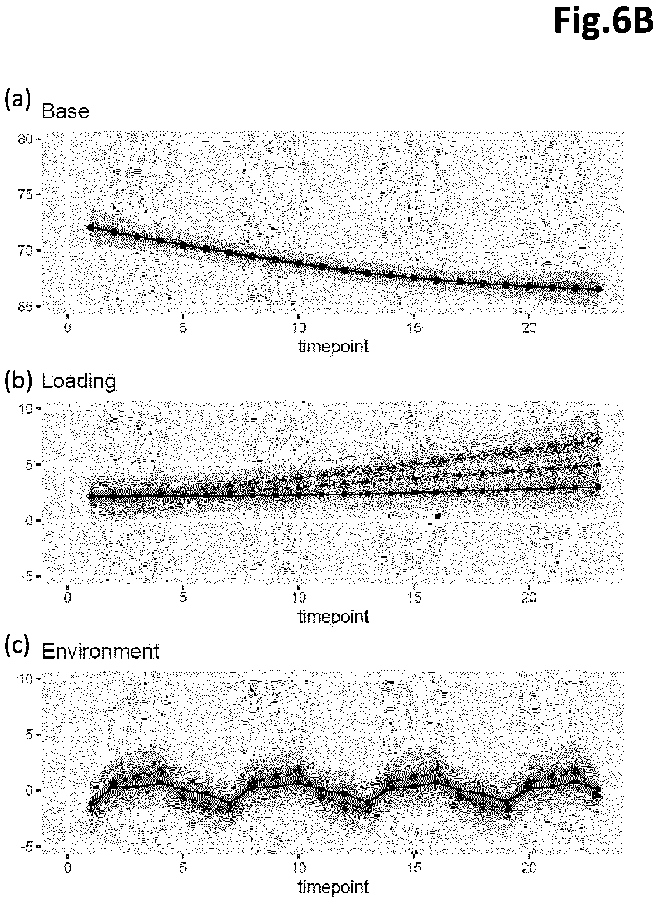

Fig. 6B illustrates results for the HR (heart rate) index obtained from heart rate variability. Fig. 6B is a graph illustrating each derived component. Fig. 6B(a) is a graph illustrating the third component (Base), Fig. 6B(b) is a graph illustrating the first component (Loading), and Fig. 6B(c) is a graph illustrating the second component (Environment). A black circle (•) indicates the result under the T26-26 condition, a black square (■) indicates the result under the T26-31 condition, a white diamond (◇) indicates the result under the T26-36 condition, and a black triangle (·) indicates the result under the T21-36 condition. Light grey represents the 95% Bayes credit interval and dark grey represents the 50% Bayes credit interval.

As can be seen from Fig. 6B(b), in the first component, an increase in the heart rate (accumulation of load on the body) can be expressed. The degree of the accumulation of the load is

$$T26-31 \; < \; T21-36 \; < \; T26-36.$$

It can be seen that the load tends to accumulate more in the T21-36 condition than in the T26-36 condition where the temperature difference is the largest. This is also consistent with the results illustrated in Fig. 6A. Such a degree of the accumulation of the load could not be found from data before analysis (not illustrated). By the technique of the present invention, the degree of the accumulation of the load can be visualized because the first component can be distinguished from the second component and the third component and derived.

As can be seen from Fig. 6B(c), the effects of the environment for each cycle are expressed in the second component. Such effects of the environment for each cycle could not be clearly found from the data before analysis. By the technique of the present invention, it is possible to distinguish and derive the second component from the first component and the third component, and thus, it is possible to visualize the effects of the environment for each cycle.

(3. HF)

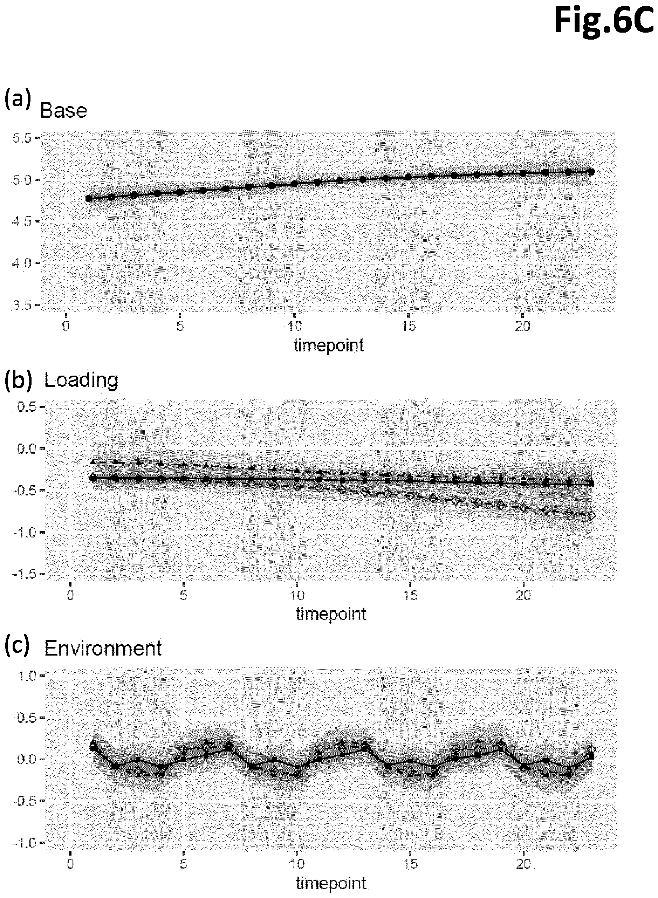

Fig. 6C illustrates results for the HF (high frequency) index obtained from heart rate variability. Fig. 6C is a graph illustrating each derived component. Fig. 6C(a) is a graph illustrating the third component (Base), Fig. 6C(b) is a graph illustrating the first component (Loading), and Fig. 6C(c) is a graph illustrating the second component (Environment). A black circle (•) indicates the result under the T26-26 condition, a black square (■) indicates the result under the T26-31 condition, a white diamond (O) indicates the result under the T26-36 condition, and a black triangle (·) indicates the result under the T21-36 condition. Light grey represents the 95% Bayes credit interval and dark grey represents the 50% Bayes credit interval.

As can be seen from Fig. 6C(b), in the first component, decrease in parasympathetic nerve activity (accumulation of load on the body) can be expressed. The degree of the accumulation of the load (the degree of the accumulation of the load is higher when the degree of the accumulation of the load is lower) is

$$T21-36 \; \leq \; T26-31 \; < \; T26-36.$$

It can be seen that the load tends to accumulate more in the T21-36 condition than in the T26-36 condition where the temperature difference is the largest. Rather, it can be seen that the load under the T21-36 condition where the temperature difference is the largest falls within the range of the T26-31 condition where the temperature difference is small. This is also consistent with the results illustrated in Fig. 6A. Such a degree of the accumulation of the load could not be found from data before analysis (not illustrated). By the technique of the present invention, the degree of the accumulation of the load can be visualized because the first component can be distinguished from the second component and the third component and derived.

As can be seen from Fig. 6C(c), the effects of the environment for each cycle are expressed in the second component. Such effects of the environment for each cycle could not be clearly found from the data before analysis. By the technique of the present invention, it is possible to distinguish and derive the second component from the first component and the third component, and thus, it is possible to visualize the effects of the environment for each cycle.

(4. Skin temperature)

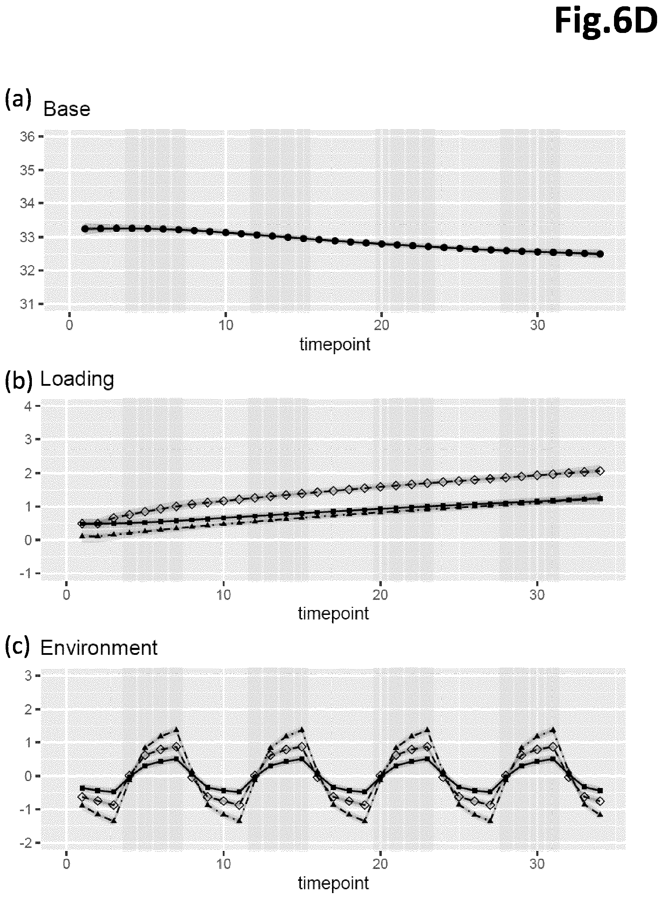

Fig. 6D illustrates the results for the skin temperature index. Fig. 6D is a graph illustrating each derived component. Fig. 6D(a) is a graph illustrating the third component (Base), Fig. 6D(b) is a graph illustrating the first component (Loading), and Fig. 6D(c) is a graph illustrating the second component (Environment). A black circle (•) indicates the result under the T26-26 condition, a black square (■) indicates the result under the T26-31 condition, a white diamond (O) indicates the result under the T26-36 condition, and a black triangle (·) indicates the result under the T21-36 condition. Light grey represents the 95% Bayes credit interval and dark grey represents the 50% Bayes credit interval.

As can be seen from Fig. 6D(b), in the first component, an increase in skin temperature (accumulation of load on the body) can be expressed. The degree of the accumulation of the load is

$$T21\text{-}36 \leq T26\text{-}31 < T26\text{-}36.$$

It can be seen that the load tends to accumulate more in the T21-36 condition than in the T26-36 condition where the temperature difference is the largest. Rather, it can be seen that the load under the T21-36 condition where the temperature difference is the largest falls within the range of the T26-31 condition where the temperature difference is small. This is also consistent with the results illustrated in Fig. 6A. Such a degree of the accumulation of the load could not be found from data before analysis (not illustrated). By the technique of the present invention, the degree of the accumulation of the load can be visualized because the first component can be distinguished from the second component and the third component and derived.

As can be seen from Fig. 6D(c), the effects of the environment for each cycle are expressed in the second component. The T21-36 condition with the largest temperature difference has the largest variation. Such effects of the environment for each cycle could not be clearly found from the data before analysis. By the technique of the present invention, it is possible to distinguish and derive the second component from the first component and the third component, and thus, it is possible to visualize the effects of the environment for each cycle.

(5. Physical fatigue)

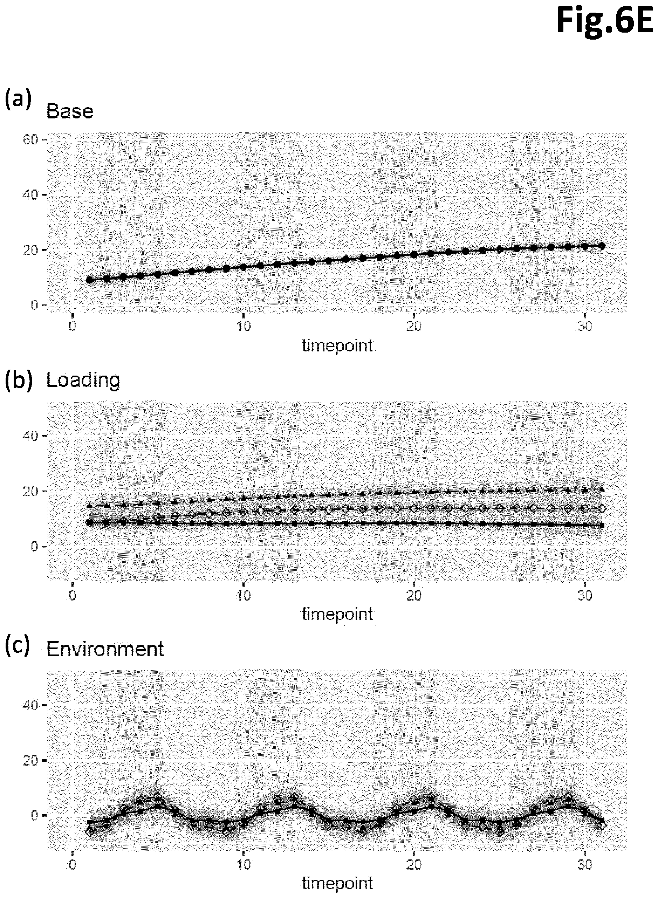

Fig. 6E illustrates the results for the physical fatigue index, which is a psychological index. Fig. 6E is a graph illustrating each derived component. Fig. 6E(a) is a graph illustrating the third component (Base), Fig. 6E(b) is a graph illustrating the first component (Loading), and Fig. 6E(c) is a graph illustrating the second component (Environment). A black circle (•) indicates the result under the T26-26 condition, a black square (■) indicates the result under the T26-31 condition, a white diamond (◇) indicates the result under the T26-36 condition, and a black triangle (·) indicates the result under the T21-36 condition. Light grey represents the 95% Bayes credit interval and dark grey represents the 50% Bayes credit interval.

As can be seen from Fig. 6E(b), the accumulation of the load on the body can be expressed in the first component. The degree of the accumulation of the load is

$$T26\text{-}31 < T26\text{-}36 < T21\text{-}36.$$

The T21-36 condition with the largest temperature difference has a large accumulation of subjective fatigue.

When Figs. 6A to 6D are compared with Fig. 6E, it can be seen that the subjective fatigue accumulation is large in the T21-36 condition having the largest temperature difference, but the accumulation of the actual load on the body is large in the T26-36 condition. That is, it is illustrated that the accumulation of subjective fatigue is not necessarily correct, and a load is actually accumulated on the body in a condition in which the subject is not fatigued. As described above, according to the technique of the present invention, it is particularly advantageous in that the deviation between the subjective accumulation of fatigue and the actual accumulation of load on the body can be clarified.

(Example 2)

Using the data measured in the temperature difference test of Example 1, prediction utilizing Bayesian statistical modeling was performed. In this example, the first component (Load), the second component (Environment), and the third component (Base) were modeled using the data up to the third round among the data measured when the test chamber A and the test chamber B were reciprocated four times. The obtained model was used to predict the fourth round trip data.

When the data of the T26-26 condition (that is, the control condition) was defined as $Base + \varepsilon_1$ and the data of each of the T26-31 condition, the T26-36 condition, and the T21-36 condition was defined as $Base + Load_n + Env_n + \varepsilon_n$ using the error terms $s_n \sim normal(0, \sigma_n)$, the model formula based on the data up to the third round trip was as follows:

[Math 9]

$$Base_t = 2Base_{t-1} - Base_{t-2} + \varepsilon_{Base,\,t-2}$$
$$\varepsilon_{Base,\,t} \sim normal\,(0,\ \sigma_{Base})$$
$$Load_{t,\,n} = 2Load_{t-1,\,n} - Load_{t-2,\,n} + \varepsilon_{Load,\,t-2}$$
$$\varepsilon_{Load,\,t} \sim cauchy\,(0,\ \sigma_{Load})$$
$$Env_{t,\,n} = -\sum_{t=1}^{N-1} Env_t + \varepsilon_{Env,\,t}$$
$$\varepsilon_{Env,\,t} \sim normal\,(0,\ \sigma_{Env})$$

Here, X-normal($\mu$, $\sigma$) means that X follows a normal distribution with an average $\mu$ and a standard deviation $\sigma$, and X~cauchy($\mu$, $\sigma$) means that X follows a cauchy distribution with an average $\mu$ and a standard deviation $\sigma$. t represents a time point, and N represents a cycle of temperature change (here, the number of time points for one cycle. For example, six time points).

When the data of the T26-26 condition (that is, the control condition) was defined as Base + $\varepsilon_1$ and the respective data of the T26-31 condition, the T26-36 condition, and the T21-36 condition were defined as Base + $Load_n$ + $Env_n$ + $\varepsilon_n$ using the error terms $\varepsilon_n$~normal (0, $\sigma_n$), the prediction model formula of the fourth round trip was as follows:

[Math 10]

$$Base_t = 2Base_{t-1} - Base_{t-2} + \varepsilon_{Base,\,t-2}$$
$$\varepsilon_{Base,\,t} \sim normal\,(0,\ \sigma_{Base})$$
$$Load_{t,\,n} = 2Load_{t-1,\,n} - Load_{t-2,\,n} + \varepsilon_{Load,\,t-2}$$
$$\varepsilon_{Load,\,t} \sim normal\,(0,\ \sigma_{Load})$$
$$Env_{t,\,n} = -\sum_{t=1}^{N-1} Env_t + \varepsilon_{Env,\,t}$$
$$\varepsilon_{Env,\,t} \sim normal\,(0,\ \sigma_{Env})$$

The estimated values obtained by the model formulas up to the third round were input to this model formula, and the change in the fourth round was predicted. Changes in LF/HF, HR, HF, skin temperature, and VAS at the fourth round were predicted.

(Results)

(1. LF/HF)

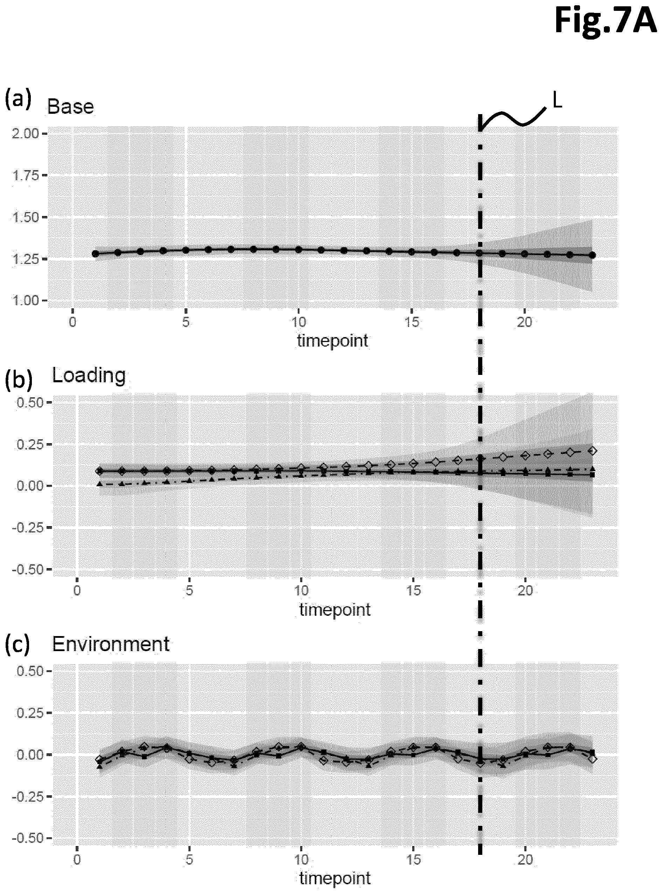

Fig. 7A illustrates results for the LF/HF index obtained from heart rate variability. Fig. 7A is a graph illustrating each derived component. Fig. 7A(a) is a graph illustrating a third component (Base), Fig. 7A(b) is a graph illustrating a first component (Loading), and Fig. 7A(c) is a graph illustrating a second component (Enviroinment). A black circle (•) indicates the result under the T26-26 condition, a black square (■) indicates the result under the T26-31 condition, a white diamond (O) indicates the result under the T26-36 condition, and a black triangle (·) indicates the result under the T21-36 condition. Light grey represents the 95% Bayes credit interval and dark grey represents the 50% Bayes credit interval. The left side of the line L is the estimated value from the actually obtained data, and the right side of the line L is the predicted value.

The predicted values were roughly consistent with the estimates from the actually obtained data illustrated in Fig. 6A. Therefore, it is considered that prediction can be performed with sufficient accuracy.

As described above, since it is possible to predict at which point in the future LF/HF, which is an index related to the overactivity of the sympathetic nerve, exceeds the threshold in a specific environment, it is advantageous in that it is possible to control the environment so as to suppress the accumulation of the load in advance.

(2. HR)

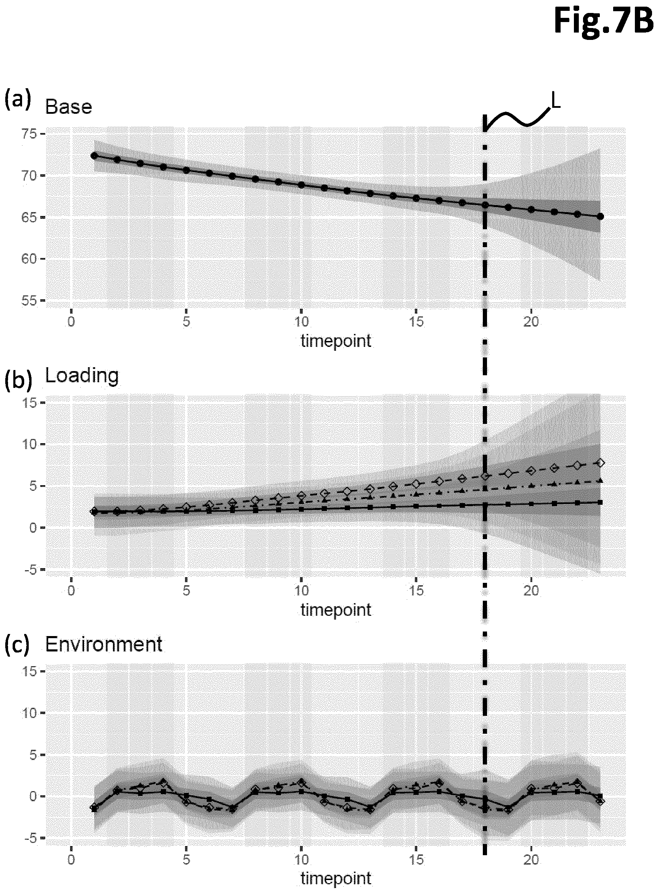

Fig. 7B illustrates the results for the HR index obtained from heart rate variability. Fig. 7B is a graph illustrating each derived component. Fig. 7B(a) is a graph illustrating the third component (Base), Fig. 7B(b) is a graph illustrating the first component (Loading), and Fig. 7B(c) is a graph illustrating the second component (Environment). A black circle (•) indicates the result under the T26-26 condition, a black square (■) indicates the result under the T26-31 condition, a white diamond (◇) indicates the result under the T26-36 condition, and a black triangle (·) indicates the result under the T21-36 condition. Light grey represents the 95% Bayes credit interval and dark grey represents the 50% Bayes credit interval. The left side of the line L is the estimated value from the actually obtained data, and the right side of the line L is the predicted value.

The predicted values were roughly consistent with the estimated values from the actually obtained data illustrated in Fig. 6B. Therefore, it is considered that prediction can be performed with sufficient accuracy.

As described above, since it is possible to predict at which point in the future the heart rate, which is an index of the load on the body, exceeds the threshold in a specific environment, it is advantageous in that it is possible to control the environment so as to prevent an excessive increase or decrease in the heart rate in advance.

(3. HF)

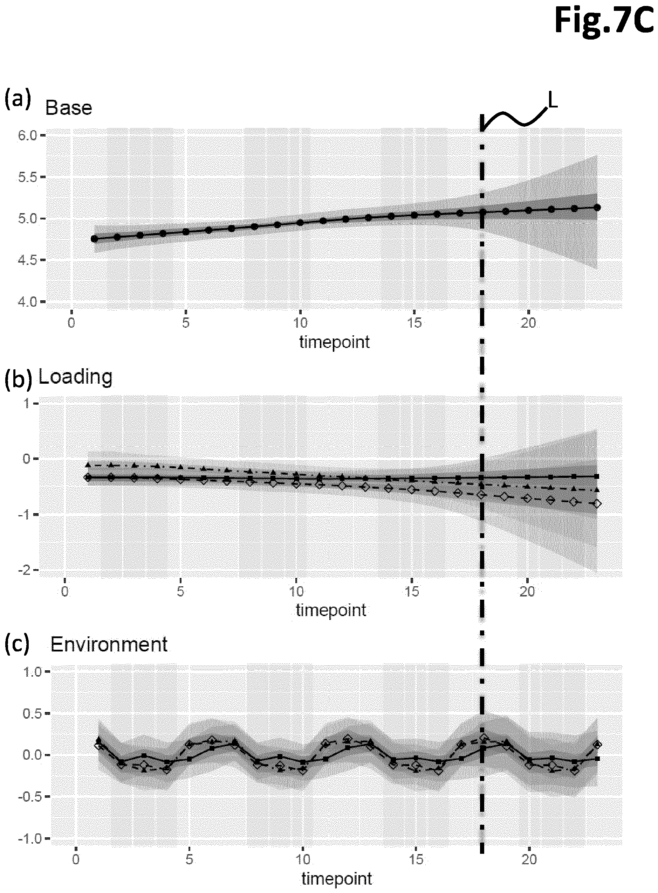

Fig. 7C illustrates results for the HF index obtained from heart rate variability. Fig. 7C is a graph illustrating each derived component. Fig. 7C(a) is a graph C illustrating the third component, Fig. 7C(b) is a graph illustrating the first component, and Fig. 7c(c) is a graph illustrating the second component. The left side of the line L is the estimated value from the actually obtained data, and the right side of the line L is the predicted value.

The predicted values were roughly consistent with the estimates from the actually obtained data illustrated in Fig. 6C. Therefore, it is considered that prediction can be performed with sufficient accuracy.

As described above, since it is possible to predict at which point in the future HF, which is an index of parasympathetic activity, exceeds the threshold in a specific environment, it is advantageous in that it is possible to control the environment so as to preliminarily suppress a decrease in HF.

(4. Skin temperature)

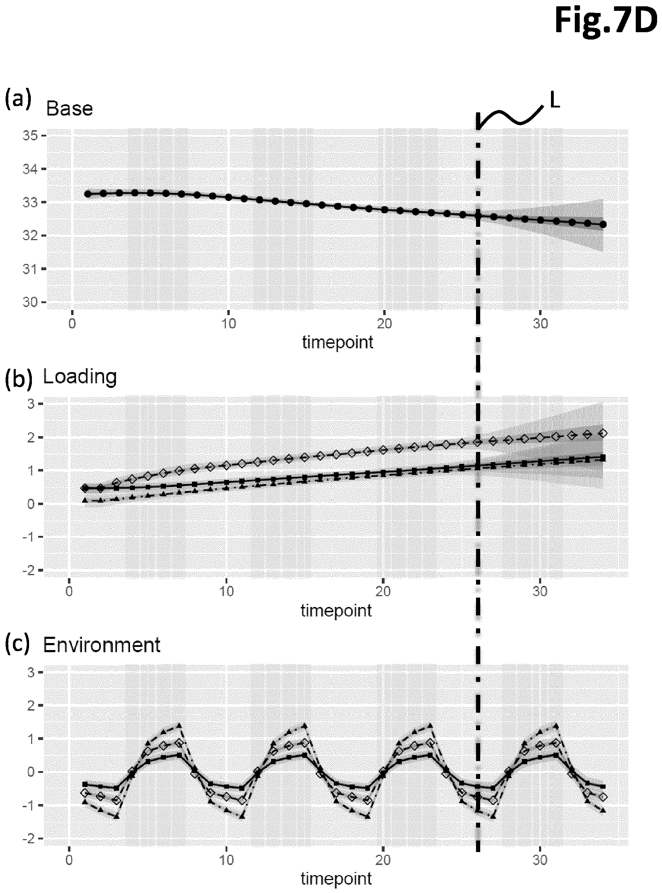

Fig. 7D illustrates the results for the skin temperature index. Fig. 7D is a graph illustrating each derived component. Fig. 7D(a) is a graph illustrating the third component, Fig. 7D(b) is a graph illustrating the first component, and Fig. 7D(c) is a graph illustrating the second component. The left side of the line L is the estimated value from the actually obtained data, and the right side of the line L is the predicted value.

The predicted values were roughly consistent with the estimates from the actually obtained data illustrated in Fig. 6D. Therefore, it is considered that prediction can be performed with sufficient accuracy.

In this way, it is possible to predict at which time point the skin temperature, which is an index of the thermal stress accumulated in the body, exceeds the threshold in a specific environment in the future. Therefore, it is advantageous in that it is possible to control the environment so as to suppress excessive variation of the body temperature in advance.

(5. Physical fatigue)

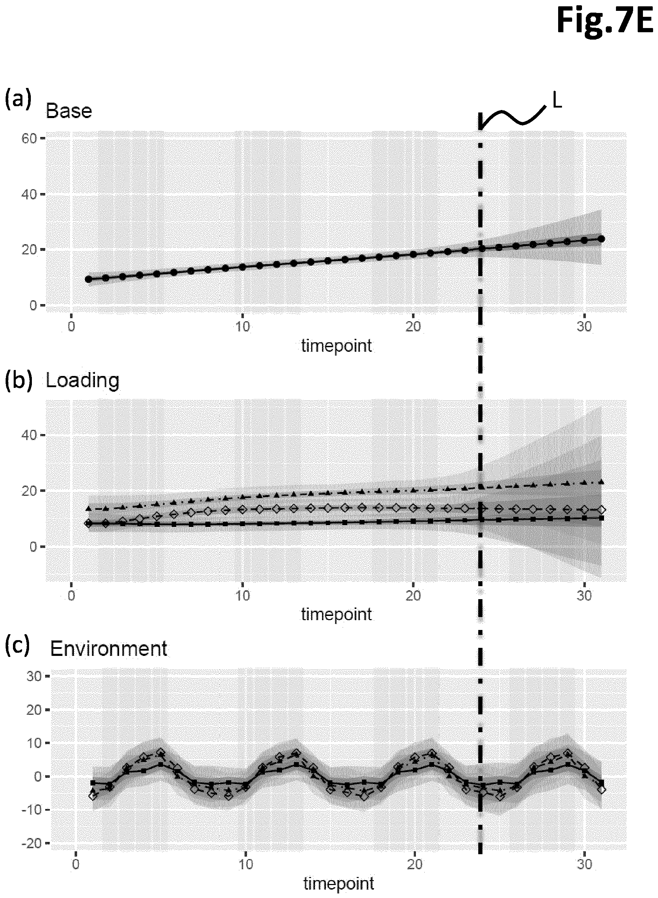

Fig. 7E illustrates the results for the physical fatigue index, which is a psychological index. Fig. 7E is a graph illustrating each derived component. Fig. 7E(a) is a graph illustrating the third component, Fig. 7E(b) is a graph illustrating the first component, and Fig. 7E(c) is a graph illustrating the second component. The left side of the line L is the estimated value from the actually obtained data, and the right side of the line L is the predicted value.

The predicted values were roughly consistent with the estimates from the actually obtained data illustrated in Fig. 6E. Therefore, it is considered that prediction can be performed with sufficient accuracy.

Since it is possible to predict at which time point such a subjective fatigue index exceeds the threshold in the future, it is possible to control the environment so as to suppress an increase in subjective fatigue.

The discrepancy between the subjective accumulation of fatigue and the actual accumulation of load on the body found in Figs. 6A to 6E was also seen in Figs. 7A to 7E.

(Example 3)

The same analysis as in Example 1 was performed on data measured on one subject among the data measured in the temperature difference test of Example 1. HR was analyzed.

(Results)

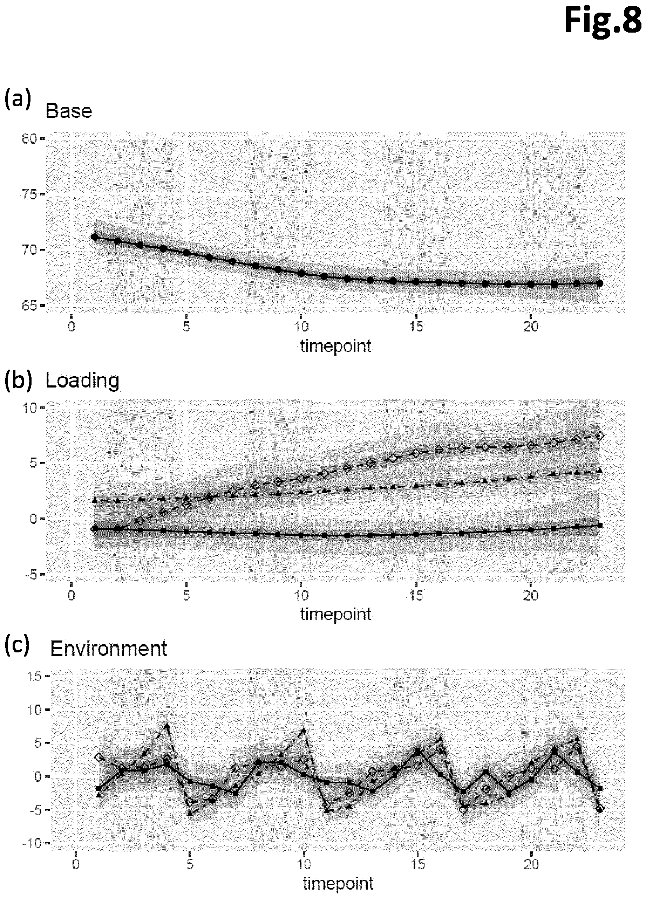

Fig. 8 is a graph illustrating each derived component. Fig. 8(a) is a graph illustrating the third component, Fig. 8(b) is a graph illustrating the first component, and Fig. 8(c) is a graph illustrating the second component.

Even with the data measured on one subject, three components could be derived similarly to the data measured on 27 subjects of Example 1, and the tendency of these components was consistent with the result of Example 1. It can be seen that the present technique can be applied to at least one subject.

(Example 4)

The same prediction as in Example 2 was performed on data measured on one subject among the data measured in the temperature difference test of Example 1. Predictions were made for HR.

(Results)

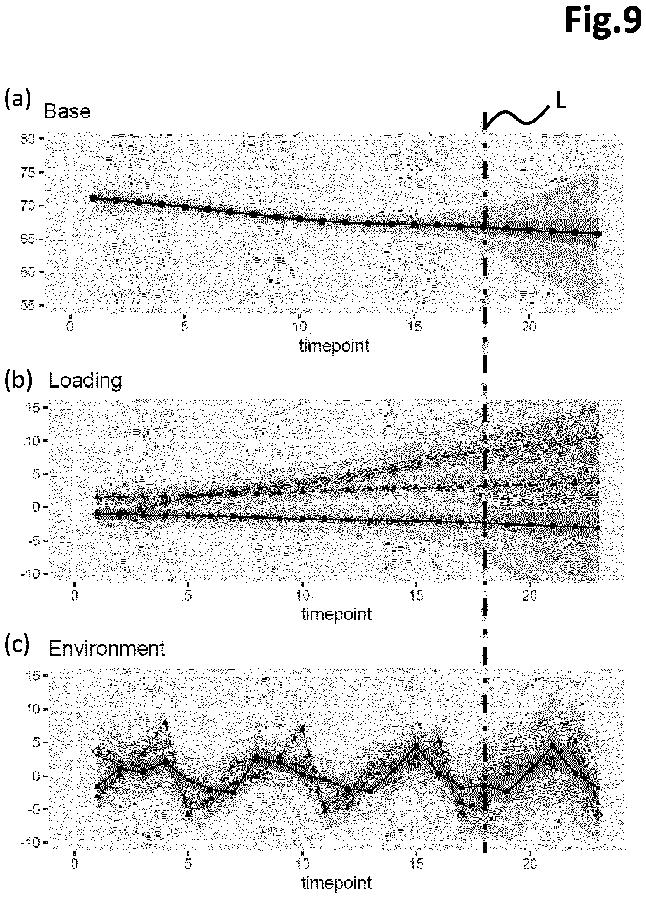

Fig. 9 is a graph illustrating each derived component. Fig. 9(a) is a graph illustrating the third component, Fig. 9(b) is a graph illustrating the first component, and Fig. 9(c) is a graph illustrating the second component. The left side of the line L is the estimated value from the actually obtained data, and the right side of the line L is the predicted value.

The predicted values were roughly consistent with the estimated values from the actually obtained data illustrated in Fig. 8. Therefore, it is considered that prediction can be performed with sufficient accuracy.

Even with the data measured on one subject, the future variations of the three components could be predicted as with the data measured on 27 subjects of Example 2. It can be seen that the present technique can be applied to at least one subject.

(Example 5)

In order to show the validity of the present invention technique for more flexible environmental changes, verification was performed on whether estimation/prediction can be correctly performed using virtual data assuming a real environment.

(Example 5A)

In the present example, it is assumed that a condition that a single event of "splashing with water" occurs when the resident stays in an outdoor area in the summer season, virtual data regarding the heart rate variability in that case is created, and the technique of the present invention is applied to the virtual data.

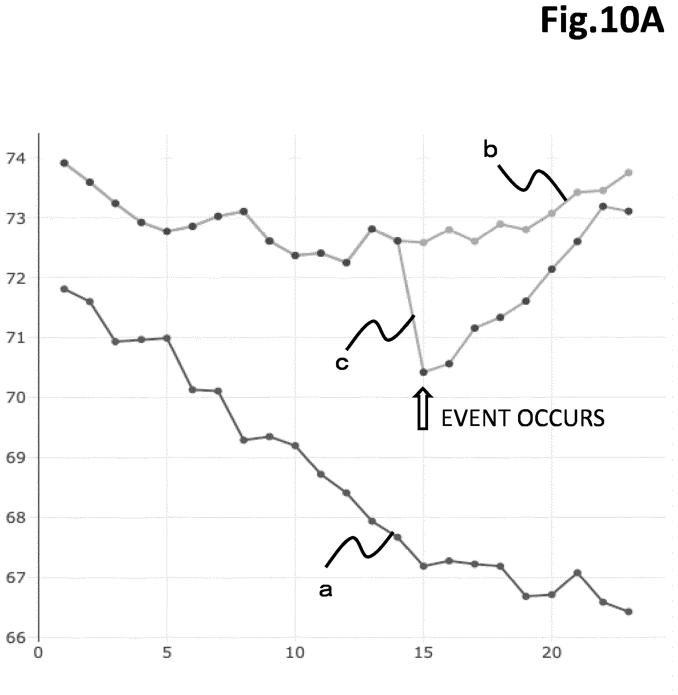

Fig. 10A illustrates a graphical representation of the created virtual data. The graph in a illustrates data regarding the heart rate variability while staying in a room temperature environment (26°C). The graph of b illustrates data regarding the heart rate variability when staying in a hot outdoor environment (36°C) in the summer season. The graph of c is data regarding the heart rate variability when a single event (for example, "cover with water") occurs while the user stays in a hot outdoor environment (36°C) in the summer season.

The observation equations $Y_a$, $Y_b$, and $Y_c$ of the data a, b, and c may be expressed, respectively, as follows:

$$Y_a = \text{Base} + \varepsilon_a$$

$$Y_b = \text{Base} + \text{Load} + \varepsilon_b$$

$Y_c$ = Base + Load + Env + $\varepsilon_c$ Here, $\varepsilon_n \sim$ normal $(0, \sigma_n^2)$ and Env = Event are set.

The state equations may be expressed, respectively, as follows:

$$\text{Base}_t = 2\text{Base}_{t-1} - \text{Base}_{t-2} + \varepsilon_{\text{Base},t-2}$$

$$\varepsilon_{\text{Base},t} \sim \text{normal}(0, \sigma_{\text{Base}})$$

$$\text{Load}_t = 2\text{Load}_{t-1} - \text{Load}_{t-2} + \varepsilon_{\text{Load},t-2}$$

$$\varepsilon_{\text{Load},t} \sim \text{normal}(0, \sigma_{\text{Load}})$$

$$\text{Env}_t = \text{Env}_{t-1} + \varepsilon_{\text{nv},t}$$

$$\varepsilon_{Env,t} \sim cauchy(0, \sigma_{Env})$$

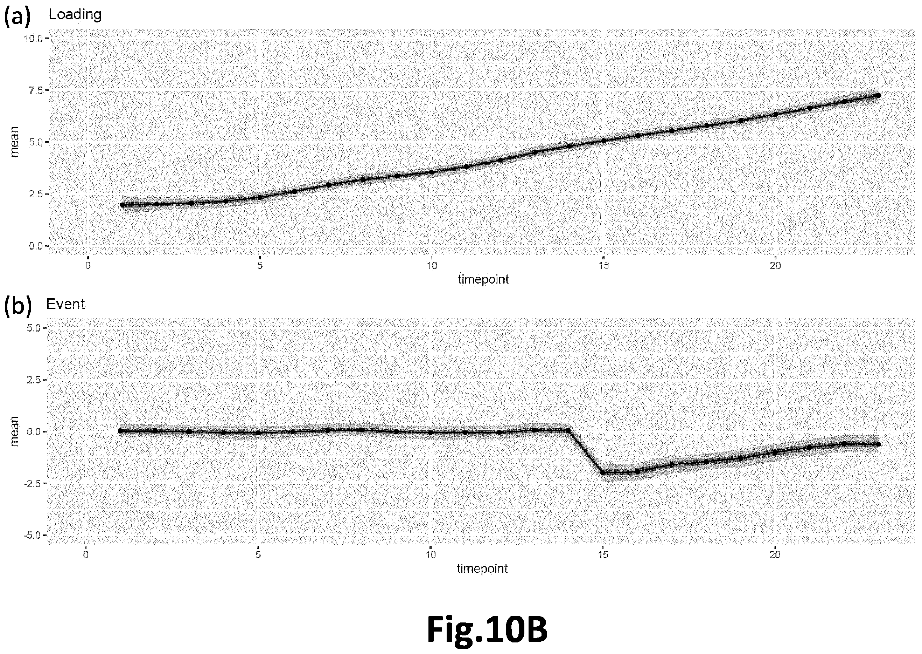

When a mathematical model having the first component, the second component, and the third component expressed by these state equations is applied to the virtual data, each component is derived as illustrated in Fig. 10B.

Fig. 10B(a) illustrates the first component (Loading) derived from the virtual data, and Fig. 10B(b) illustrates the second component (Event) derived from the virtual data.

It can be seen from Fig. 10B(a) that the accumulation of the load due to the hot environment can be derived. Furthermore, it can be seen from Fig. 10B(b) that a change due to a single event can be grasped.

As described above, according to the technique of the present invention, the first component and the second component can be derived even in a case where a single event occurs.

(Example 5B)

In the present example, assuming a condition that a person goes back and forth between a hot outdoor area and a cool indoor area at a random time and a random period, virtual data regarding the heart rate variability in this case is created, and the technique of the present invention is applied to the virtual data.

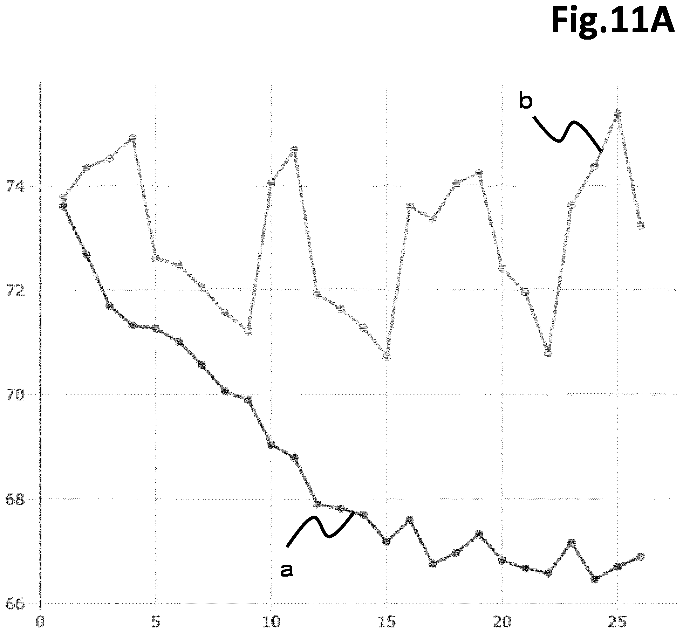

Fig. 11A illustrates a graphical representation of the created virtual data. The graph of a illustrates virtual data regarding the heart rate variability while continuing to stay in a room temperature environment (26°C). The graph in b illustrates virtual data regarding the heart rate variability when moving between a cool indoor room temperature environment (26°C) and a hot outdoor hot environment (36°C).

The observation equations $Y_a$ and $Y_b$ of the data a and b may be expressed, respectively, as follows:

$$Y_a = Base + \varepsilon_a$$

$$Y_b = Base + Load + Env + \varepsilon_b \text{ Here, } \varepsilon n \sim normal(0, \sigma_n^2).$$

$$Env = Event * Onset$$

Onset is a binary representation of the presence or absence of an event according to the time point to be analyzed. For example, Onset = [0, 0, 0, 1, 1, 1, 0, 0, 0,......].

The state equations may be expressed, respectively, as follows:

$$Base_t = 2Base_{t-1} - Base_{t-2} + \varepsilon_{Base,t-2}$$

$$\varepsilon_{Base,t} \sim normal(0, \sigma_{Base})$$

$$Load_t = 2Load_{t-1} - Load_{t-2} + \varepsilon_{Load,t-2}$$

$$\varepsilon_{Load,t} \sim cauchy(0, \sigma_{Load})$$

$$Event_t = Event_{t-1} + \varepsilon_{Event,t}$$

$$\varepsilon_{Event,t} \sim normal(0, \sigma_{Event}).$$

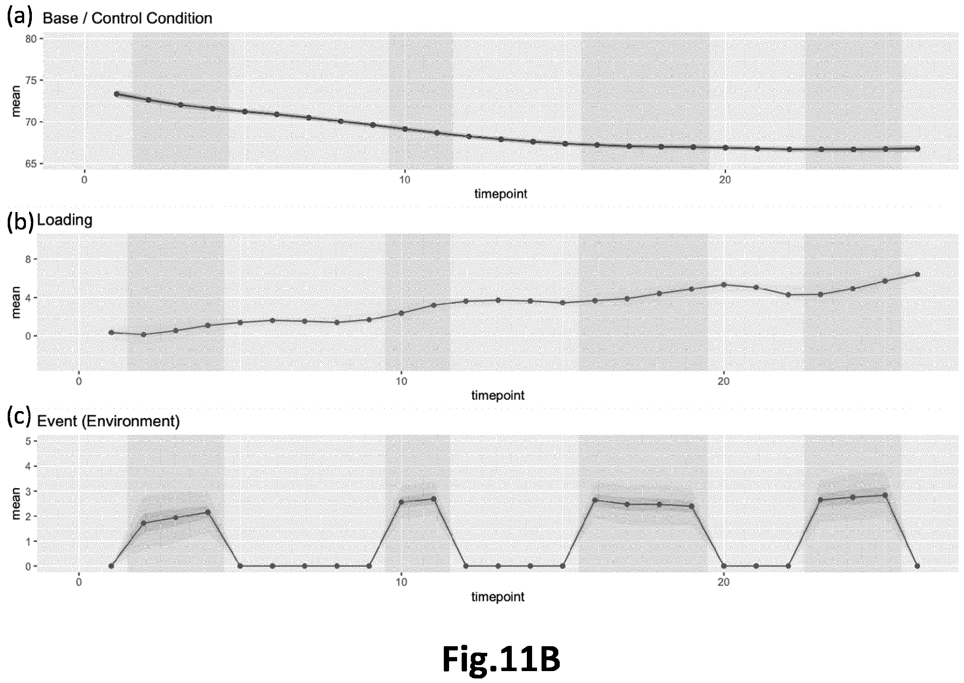

When a mathematical model having the first component, the second component, and the third component expressed by these state equations is applied to the virtual data, each component is derived as illustrated in Fig. 11B.

Fig. 11B(a) illustrates the third component (Base) derived from the virtual data, Fig. 11B(b) illustrates the first component (Loading) derived from the virtual data, and Fig. 11B(c) illustrates the second component (Environment) derived from the virtual data.

It can be seen from Fig. 11B(b) that the accumulation of the load due to traffic between the room temperature environment and the hot environment can be derived. Furthermore, it can be seen from Fig. 11B(c) that a change due to an event occurring at random timing in a random period can be grasped.

As described above, according to the technique of the present invention, the first component and the second component can be derived even in a case where an arbitrary event having no regularity occurs.

INDUSTRIAL APPLICABILITY

The present invention is useful as providing a system or the like for quantifying the effects of a change in state regarding a subject during a predetermined time period.

DESCRIPTION OF REFERENCE SIGNS

100, 100' system
110 reception means
120 derivation means
130 output means
140 alarm activation means
150 control means
160 estimation/prediction means

**Claims**

1. A system for quantifying effects of a change in state during a predetermined time period regarding a subject, the change occurring during the predetermined time period including at least a change from a first state to a second state, the system comprising:

    a reception means that receives data measured on the subject, the data including at least first data measured on the subject in the first state and second data measured on the subject in the second state;
    a derivation means that derives, from the received data, at least a first component representing accumulation of a load on the subject during the predetermined time period and a second component varying following the change; and
    an output means that outputs at least one of the derived first component and second component.

2. The system according to claim 1, wherein the derivation means derives the first component and the second component from the received data by applying, to the received data, a mathematical model including the first component and the second component as components.

3. The system according to claim 2, wherein

    the mathematical model further includes a third component that does not depend on the change as a component, and
    the derivation means derives the first component, the second component, and the third component from the received data by applying the mathematical model to the received data.

4. The system according to claim 3, wherein the third component is based on data measured on the subject or another subject during the predetermined time period in a case where the change does not exist.

5. The system according to any one of claims 2 to 4, wherein, for each component of the mathematical model, a time series variation of each component is expressed using a local variation that follows a predetermined distribution.

6. The system according to claim 5, wherein the predetermined distribution includes at least one of a normal distribution, a Cauchy distribution, a binomial distribution, a Poisson distribution, a multinomial distribution, and a Bernoulli distribution.

7. The system according to any one of claims 2 to 6, wherein

    the reception means further receives change information defining a change from the first state to the second state, and
    the second component of the mathematical model is modeled based on the change information.

8. The system according to claim 7, wherein

the state regarding the subject includes a state of an environment of the subject,
a change in the state of the environment of the subject is a periodic change or a change related to an event, and
the change information includes a period of the change or a timing of the change.

9. The system according to any one of claims 1 to 8, wherein

the state regarding the subject includes a state of an environment of the subject, and
a change in the state of the environment of the subject is at least one of a change in temperature, a change in humidity, a change in radiation, a change in air quality, a change in airflow.

10. The system according to claim 7 or 8, wherein the state regarding the subject includes a state of activity of the subject,

a change in the state of the activity of the subject is a periodic change or a change related to an event, and
the change information includes a period of the change or a timing of the change.

11. The system according to any one of claims 1 to 10, wherein

the state regarding the subject includes a state of activity of the subject, and
a change in the state of the activity of the subject is at least one of a change in state regarding clothing, a change in state regarding working, a change in state regarding exercise, a change in state regarding sleep, a change in state regarding dietary, and a change in state regarding excretion.

12. The system according to any one of claims 1 to 11, wherein

the change occurring during the predetermined time period further includes a change from the second state to a third state, and
the data further includes third data measured on the subject in the third state.

13. The system according to claims 1 to 12, wherein the data includes at least one of an autonomic nervous index based on a heart rate or heart rate variability, a skin temperature, a sweating index, a blood pressure, or a psychological assessment value.

14. The system according to any one of claims 1 to 13, further comprising an alarm activation means that activates an alarm when the output first component and/or second component exceeds a predetermined threshold.

15. The system according to any one of claims 1 to 14, further comprising an estimation/prediction means that estimates or predicts an index of a load on the subject due to the change, based on at least the output first component and/or second component.

16. The system according to claim 15, wherein the estimation/prediction means predicts future variations of the first component and/or the second component.

17. The system according to any one of claims 1 to 16, further comprising a control means that controls an environment adjustment device based on at least the output first component and/or second component.

18. The system according to claim 17, wherein
when the output first component and/or second component exceeds a predetermined threshold, the control means is configured to:

determine an environment for increasing or decreasing the first component and/or the second component; and
control the environment adjustment device to achieve the environment.

19. The system according to any one of claims 1 to 18, wherein

the first component is a component that monotonically increases or monotonically decreases in each state, and
the second component is a component that varies to approach a predetermined value in each state.

20. A system for quantifying effects of a change in state during a predetermined time period regarding a subject, the change including at least a change from a first state to a second state, the system comprising:

a reception means that receives data measured on the subject, the data including at least first data measured on the subject in the first state and second data measured on the subject in the second state;
a derivation means that, by applying, to the received data, a mathematical model including a first component, a second component, and a third component as components, derives the first component, the second component, and the third component from the received data; and
an output means that outputs the derived first component and/or second component.

21. A system for quantifying effects of a change in state during a predetermined time period regarding a subject, the change including at least a change from a first state to a second state, the system comprising:

a reception means that receives data measured on the subject, the data including at least first data measured on the subject in the first state, second data measured on the subject in the second state, and change information defining a change from the first state to the second state;
a derivation means that, by applying, to the received data, a mathematical model including a first component, a second component, and a third component as components, derives the first component, the second component, and the third component from the received data, the second component in the mathematical model being modeled based on the change information; and
an output means that outputs the derived first component and/or second component,
wherein the data includes at least one of an autonomic nervous index based on a heart rate or heart rate variability, a skin temperature, a sweating index, a blood pressure, or a psychological assessment value.

22. A method for quantifying effects of a change in state during a predetermined time period regarding a subject, the change occurring during the predetermined time period including at least a change from a first state to a second state, the method comprising:

receiving data measured on the subject, the data including at least first data measured on the subject in the first state and second data measured on the subject in the second state;
deriving, from the received data, at least a first component representing accumulation of a load on the subject during the predetermined time period and a second component varying following the change; and
outputting at least one of the derived first component and second component.

23. A program for quantifying effects of a change in state during a predetermined time period regarding a subject, the change occurring during the predetermined time period including at least a change from a first state to a second state, the program being executed in a computer including a processor unit, the program causing the processor unit to perform processing comprising:

receiving data measured on the subject, the data including at least first data measured on the subject in the first state and second data measured on the subject in the second state;
deriving, from the received data, at least a first component representing accumulation of a load on the subject during the predetermined time period and a second component varying following the change; and
outputting at least one of the derived first component and second component.

(a)

(b)

**Fig.1**

(a)

(b)

30

S

41

40

**Fig.2**

**Fig.3**

**Fig.4A**

**Fig.4B**

100"

110          120          130

RECEPTION
MEANS

DERIVATION
MEANS

OUTPUT
MEANS

160

ESTIMATION
/PREDICTION
MEANS

**Fig.4C**

500

```
┌─────────────────────────────────────────────┐
│                                             │  S501
│        RECEIVE DATA MEASURED ON SUBJECT      │
│                                             │
└─────────────────────────────────────────────┘
                      │
                      ▼
┌─────────────────────────────────────────────┐
│   DERIVE AT LEAST FIRST COMPONENT AND SECOND │  S502
│        COMPONENT FROM RECEIVED DATA          │
│                                             │
└─────────────────────────────────────────────┘
                      │
                      ▼
┌─────────────────────────────────────────────┐
│      OUTPUT DERIVED FIRST COMPONENT AND/OR   │  S503
│            SECOND COMPONENT                  │
│                                             │
└─────────────────────────────────────────────┘
```

**Fig.5**

Fig.6A

# Fig.6B

(a) Base

(b) Loading

(c) Environment

# Fig.6C

(a) Base

(b) Loading

(c) Environment

# Fig.6D

(a) Base

(b) Loading

(c) Environment

# Fig.6E

(a) Base

(b) Loading

(c) Environment

**Fig.7A**

# Fig.7B

(a) Base

(b) Loading

(c) Environment

**Fig.7C**

(a) Base

(b) Loading

(c) Environment

# Fig.7D

(a) Base

(b) Loading

(c) Environment

# Fig.7E

(a) Base

(b) Loading

(c) Environment

# Fig.8

(a) Base

(b) Loading

(c) Environment

# Fig.9

(a) Base

(b) Loading

(c) Environment

# Fig.10A

(a) Loading

(b) Event

**Fig.10B**

**Fig.11A**

(a) Base / Control Condition

(b) Loading

(c) Event (Environment)

**Fig.11B**

EP 4 471 800 A1

EP 4 471 800 A1

# INTERNATIONAL SEARCH REPORT

International application No.

**PCT/JP2023/002729**

## A. CLASSIFICATION OF SUBJECT MATTER

***G16H 50/30***(2018.01)i
FI:   G16H50/30

According to International Patent Classification (IPC) or to both national classification and IPC

## B. FIELDS SEARCHED

Minimum documentation searched (classification system followed by classification symbols)

G16H10/00-80/00;

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| X | JP 2012-030038 A (SONY CORP.) 16 February 2012 (2012-02-16) paragraphs [0063]-[0069], [0075], [0076], [0089]-[0093], [0101]-[0119], fig. 3 | 1-23 |

☐ Further documents are listed in the continuation of Box C.          ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **01 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

57

## INTERNATIONAL SEARCH REPORT
### Information on patent family members

International application No.

**PCT/JP2023/002729**

| Patent document cited in search report | | | Publication date (day/month/year) | Patent family member(s) | Publication date (day/month/year) |
|---|---|---|---|---|---|
| JP | 2012-030038 | A | 16 February 2012 | US 2013/0110409 A1<br>paragraphs [0083]-[0089],<br>[0096], [0097], [0110]-[0114],<br>[0122]-[0144], fig. 3<br>CN 113113077 A<br>paragraphs [0091]-[0096],<br>[0106], [0107], [0121]-[0125],<br>[0133]-[0161], fig. 3 | |

Form PCT/ISA/210 (patent family annex) (January 2015)

## EP 4 471 800 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017038839 A